# EUROPEAN PATENT SPECIFICATION

(11) **EP 2 669 375 B1**
(45) Date of publication and mention of the grant of the patent: **17.10.2018**
(21) Application number: 12739392.4
(22) Date of filing: 19.01.2012
(51) Int. Cl.: C12N 1/19, C12P 21/00, C07K 16/24, C12N 15/09

(54) **YEAST OF THE GENUS PICHIA MODIFIED TO EXPRESS HIGH LEVELS OF A MPP1 HOMOLOG AND PROCESS FOR PRODUCING PROTEIN**
ZUR EXPRESSION VON ERHÖHTEN MENGEN AN MPP1 HOMOLOG MODIFIZIERTE HEFE DER GATTUNG PICHIA UND VERFAHREN ZUR HERSTELLUNG VON PROTEIN
LEVURE DU GENRE, PICHIA, MODIFIÉE POUR EXPRIMER UN NIVEAU ÉLEVÉ D'UN HOMOLOGUE DE MPP1 ET PROCÉDÉ DE PRODUCTION D'UNE PROTÉINE

(30) Priority: 27.01.2011 JP 2011015719
(43) Date of publication of application: 04.12.2013
(73) Proprietor: Kaneka Corporation, Osaka-shi, Osaka 530-8288 (JP)
(72) Inventor: NISHI Teruyuki, Takasago-shi Hyogo 676-8688 (JP); ISHIGURO Kojiro, Takasago-shi Hyogo 676-8688 (JP)
(74) Representative: Hoffmann Eitle
(86) International application number: PCT/JP2012/051102
(87) International publication number: WO 2012/102171

(56) References cited:
- EP-A1- 2 489 724
- WO-A2-2010/004042
- WO-A2-2012/129036
- JP-A- 2009 082 033
- VAN ZUTPHEN TIM ET AL: "Adaptation of Hansenula polymorpha to methanol: a transcriptome analysis", BMC GENOMICS, BIOMED CENTRAL LTD, LONDON, UK, vol. 11, no. 1, 4 January 2010 (2010-01-04), page 1, XP021066054, ISSN: 1471-2164 -& Tim Van Zutphen et al: "Supplemental Table S1", , vol. 11, no. 1 4 January 2010 (2010-01-04), XP002738971, Retrieved from the Internet: URL:http://www.biomedcentral.com/1471-2164 /11/1 [retrieved on 2015-04-27]
- ABDELMOULA-SOUISSI ET AL: "High-level expression of human tumour suppressor P53 in the methylotrophic yeast: Pichia pastoris", PROTEIN EXPRESSION AND PURIFICATION, ACADEMIC PRESS, SAN DIEGO, CA, vol. 54, no. 2, 25 May 2007 (2007-05-25), pages 283-288, XP022090900, ISSN: 1046-5928, DOI: 10.1016/J.PEP.2007.03.015
- HYUN AH KANG ET AL: "DEVELOPMENT OF EXPRESSION SYSTEMS FOR THE PRODUCTION OF RECOMBINANT HUMAN SERUM ALBUMIN USING THE MOX PROMOTER IN HANSENULA POLYMORPHA DL-1", BIOTECHNOLOGY AND BIOENGINEERING, WILEY & SONS, HOBOKEN, NJ, US, vol. 76, no. 2, 1 September 2001 (2001-09-01), pages 175-185, XP001154405, ISSN: 0006-3592, DOI: 10.1002/BIT.1157
- VAN DER KLEI I J ET AL: "The significance of peroxisomes in methanol metabolism in methylotrophic yeast", BIOCHIMICA ET BIOPHYSICA ACTA. MOLECULAR CELL RESEARCH, ELSEVIER SCIENCE PUBLISHERS, AMSTERDAM, NL, vol. 1763, no. 12, 1 December 2006 (2006-12-01), pages 1453-1462, XP027896779, ISSN: 0167-4889 [retrieved on 2006-12-01]
- HIROYA YURIMOTO ET AL: "Methanol-inducible gene expression and heterologous protein production in the methylotrophic yeast Candida boidinii", BIOTECHNOLOGY AND APPLIED BIOCHEMISTRY, vol. 53, no. 2, 1 June 2009 (2009-06-01), page 85, XP055185777, ISSN: 0885-4513, DOI: 10.1042/BA20090030
- TIM VAN ZUTPHEN ET AL.: 'Adaptation of Hansenula polymorpha to methanol: a transcriptome analysis' BMC GENOMICS vol. 11, no. 1, 2010, XP021066054
- ADRIANA NIVEA LEAO-HELDER ET AL.: 'Transcriptional Down-regulation of Peroxisome Numbers Affects Selective Peroxisome Degradation in Hansenula polymorpha' J. BIOL. CHEM. vol. 278, no. 42, 2003, pages 40749 - 40756, XP055123796
- YU SASANO ET AL.: 'Trmlp, a Zn(II)2Cys6-Type Transcription Factor, Is a Master Regulator of Methanol-Specific Gene Activation in the Methylotrophic Yeast Candida boidinii' EUKARYOT CELL vol. 7, no. 3, 2008, pages 527 - 536, XP002506159
- HIROYA YURIMOTO: 'Molecular Basis of Methanol- Inducible Gene Expression and Its Application in the Methylotrophic Yeast Candida boidinii' BIOSCIENCE, BIOTECHNOLOGY, AND BIOCHEMISTRY vol. 73, no. 4, 2009, pages 793 - 800, XP055123797
- KRISTOF DE SCHUTTER ET AL.: 'Genome sequence of the recombinant protein production host Pichia pastoris' NATURE BIOTECHNOLOGY vol. 27, no. 6, 2009, pages 561 - 566, XP008156426
- DATABASE GENBANK [Online] 20 July 2002 E. BERARDI ET AL.: 'Pichia angusta transcriptional activator Mut3p (MUT3) gene', XP003030172 Database accession no. AY046886

### SEQUENCE LISTING

<110> KANEKA Corporation
<120> Yeast for transformation and method for producing proteins
<130> B100590
<150> JP 2011-015719
   <151> 2011-1-27
<160> 52
<170> PatentIn version 3.1
<210> 1
   <211> 1511
   <212> DNA
   <213> Pichia angusta
<400> 1
   tcgacgcgga gaacgatctc ctcgagctgc tcgcggatca gcttgtggcc cggtaatgga      60
   accaggccga cggcacgctc cttgcggacc acggtggctg gcgagcccag tttgtgaacg     120
   aggtcgttta gaacgtcctg cgcaaagtcc agtgtcagat gaatgtcctc ctcggaccaa     180
   ttcagcatgt tctcgagcag ccatctgtct ttggagtaga agcgtaatct ctgctcctcg     240
   ttactgtacc ggaagaggta gtttgcctcg ccgcccataa tgaacaggtt ctctttctgg     300
   tggcctgtga gcagcgggga cgtctggacg gcgtcgatga ggcccttgag gcgctcgtag     360
   tacttgttcg cgtcgctgta gccggccgcg gtgacgatac ccacatagag gtccttggcc     420
   attagtttga tgaggtgggg caggatgggc gactcggcat cgaaattttt gccgtcgtcg     480
   tacagtgtga tgtcaccatc gaatgtaatg agctgcagct tgcgatctcg gatggttttg     540
   gaatggaaga accgcgacat ctccaacagc tgggccgtgt tgagaatgag ccggacgtcg     600
   ttgaacgagg gggccacaag ccggcgtttg ctgatggcgc ggcgctcgtc ctcgatgtag     660
   aaggcctttt ccagaggcag tctcgtgaag aagctgccaa cgctcggaac cagctgcacg     720
   agccgagaca attcgggggt gccggctttg gtcatttcaa tgttgtcgtc gatgaggagt     780
   tcgaggtcgt ggaagatttc cgcgtagcgg cgttttgcct cagagtttac catgaggtcg     840
   tccactgcag agatgccgtt gctcttcacc gcgtacagga cgaacggcgt ggccagcagg     900
   cccttgatcc attctatgag gccatctcga cggtgttcct tgagtgcgta ctccactctg     960
   tagcgactgg acatctcgag actgggcttg ctgtgctgga tgcaccaatt aattgttgcc    1020
   gcatgcatcc ttgcaccgca agtttttaaa acccactcgc tttagccgtc gcgtaaaact    1080
   tgtgaatctg gcaactgagg gggttctgca gccgcaaccg aacttttcgc ttcgaggacg    1140
   cagctggatg gtgtcatgtg aggctctgtt tgctggcgta gcctacaacg tgaccttgcc    1200
   taaccggacg gcgctaccca ctgctgtctg tgcctgctac cagaaaatca ccagagcagc    1260
   agagggccga tgtggcaact ggtggggtgt cggacaggct gtttctccac agtgcaaatg    1320
   cgggtgaacc ggccagaaag taaattctta tgctaccgtg cagcgactcc gacatcccca    1380
   gtttttgccc tacttgatca cagatggggt cagcgctgcc gctaagtgta cccaaccgtc    1440
   cccacacggt ccatctataa atactgctgc cagtgcacgg tggtgacatc aatctaaagt    1500
   acaaaaacaa a                                                          1511
<210> 2
   <211> 607
   <212> DNA
   <213> Pichia angusta
<400> 2
   ggagacgtgg aaggacatac cgcttttgag aagcgtgttt gaaaatagtt ctttttctgg      60
   tttatatcgt ttatgaagtg atgagatgaa aagctgaaat agcgagtata ggaaaattta     120
   atgaaaatta aattaaatat tttcttaggc tattagtcac cttcaaaatg ccggccgctt     180
   ctaagaacgt tgtcatgatc gacaactacg actcgtttac ctggaacctg tacgagtacc     240
   tgtgtcagga gggagccaat gtcgaggttt tcaggaacga tcagatcacc attccggaga     300
   ttgagcagct caagccggac gttgtggtga tatcccctgg tcctggccat ccaagaacag     360
   actcgggaat atctcgcgac gtgatcagcc attttaaagg caagattcct gtctttggtg     420
   tctgtatggg ccagcagtgt atcttcgagg agtttggcgg agacgtcgag tatgcgggcg     480
   agattgtcca tggaaaaacg tccactgtta agcacgacaa caagggaatg ttcaaaaacg     540
   ttccgcaaga tgttgctgtc accagatacc actcgctggc cggaacgctc aagtcgcttc     600
   cggactg                                                               607
<210> 3
   <211> 1251
   <212> DNA
   <213> Pichia angusta
<400> 3
   gagtccctga gtacgtaagc ggtttggtaa tacgaaataa aaagacagga atgagtaagt      60
   ggatggtttt tcaatttttc cggtaccggc gcaaaatagt tgcatcattt tgcaatcatg     120
   agtaagaaca ttgtgcttct ccctggtgat cacgtgggcc ccgaggttgt tgcggaggcc     180
   gtcaaggttc tcgaggctgt ctcgtcggca atcggcgtta agttcaactt ttccaagcac     240
   ctgatcggcg gtgcctcgat cgatgcttat ggggtgccat tgtccgacga ggccctcgaa     300
   gccgccaaga aggctgacgc cgttttgctt ggagccgtcg gaggacctaa gtggggaacc     360
   ggctccgtgc gtcctgagca gggtctgttg aagatcagaa aagagctcaa cttgtacgcg     420
   aacctgcgtc catgcagttt tgcttccgac gctcttctga agctatctcc actgaaatca     480
   gaaatcgtca agggcactga ctttgttgtt gtgcgtgagt tggttggtgg aatctacttt     540
   ggtgaccgca aggaggatgc cggcgacgga gttgccagcg acactgagag ctattctgtt     600
   ccagaggtgc agagaatcac gagaatggcg gcctttttgg cgctgcagag cgacccaccg     660
   ctcccactgt ggtcgctgga caaagccaac gtgcttgcat cctcgcgttt gtggcggaag     720
   actgttgagg agaccatcaa gaacgagttc ccgcagctga cggtgcagca ccagctgatc     780
   gactcggcag ccatgatttt ggtcaagtcg ccaacgaaac tcaacggtgt cattgtcacc     840
   aacaacatgt ttggcgacat catcagtgac gaggccagtg tgattcctgg gtctctgggc     900
   ctgctgcctt ctgcctcgtt ggcgtctctg ccagacacaa acaaggcgtt tggtctttac     960
   gagccctgcc acggctcggc gccagatttg ggcccgggca aggtcaatcc attggccaca    1020
   attttgtctg ccgccatgat gctgaagctg tcgctggact tggtggatgc cggccgtgcg    1080
   atcgagcagg ccgtcaagaa cgtcctggat gcaggtatca tgactgccga tttgggtgga    1140
   agctcctcaa cacaggaagt tggtgatgct gttgcgcagg aggtggccaa gctactcaag    1200
   aactaaataa gggagaaaaa aaagtaggat ctcgaataat tcctaaataa t             1251
<210> 4
   <211> 931
   <212> DNA
   <213> Pichia pastoris
<400> 4
   aacatccaaa gacgaaaggt tgaatgaaac ctttttgcca tccgacatcc acaggtccat      60
   tctcacacat aagtgccaaa cgcaacagga ggggatacac tagcagcaga ccgttgcaaa     120
   cgcaggacct ccactcctct tctcctcaac acccactttt gccatcgaaa aaccagccca     180
   gttattgggc ttgattggag ctcgctcatt ccaattcctt ctattaggct actaacacca     240
   tgactttatt agcctgtcta tcctggcccc cctggcgagg ttcatgtttg tttatttccg     300
   aatgcaacaa gctccgcatt acacccgaac atcactccag atgagggctt tctgagtgtg     360
   gggtcaaata gtttcatgtt ccccaaatgg cccaaaactg acagtttaaa cgctgtcttg     420
   gaacctaata tgacaaaagc gtgatctcat ccaagatgaa ctaagtttgg ttcgttgaaa     480
   tgctaacggc cagttggtca aaaagaaact tccaaaagtc gccataccgt ttgtcttgtt     540
   tggtattgat tgacgaatgc tcaaaaataa tctcattaat gcttagcgca gtctctctat     600
   cgcttctgaa ccccggtgca cctgtgccga aacgcaaatg gggaaacacc cgctttttgg     660
   atgattatgc attgtctcca cattgtatgc ttccaagatt ctggtgggaa tactgctgat     720
   agcctaacgt tcatgatcaa aatttaactg ttctaacccc tacttgacag caatatataa     780
   acagaaggaa gctgccctgt cttaaacctt tttttttatc atcattatta gcttactttc      840
   ataattgcga ctggttccaa ttgacaagct tttgatttta acgactttta acgacaactt     900
   gagaagatca aaaaacaact aattattcga a                                    931
<210> 5
   <211> 255
   <212> DNA
   <213> Saccharomyces cerevisiae
<400> 5
   atgagatttc cttcaatttt tactgcagtt ttattcgcag catcctccgc attagctgct      60
   ccagtcaaca ctacaacaga agatgaaacg gcacaaattc cggctgaagc tgtcatcggt     120
   tactcagatt tagaagggga tttcgatgtt gctgttttgc cattttccaa cagcacaaat     180
   aacgggttat tgtttataaa tactactatt gccagcattg ctgctaaaga agaaggggta     240
   tctctcgaga aaaga                                                      255
<210> 6
   <211> 645
   <212> DNA
   <213> Artificial
<220>
   <223> Light chain
<400> 6
   gacatccaga tgacccagtc tccatcctcc ctgtctgcat ctgtagggga cagagtcacc      60
   atcacttgtc gggcaagtca gggcatcaga aattacttag cctggtatca gcaaaaacca     120
   gggaaagccc ctaagctcct gatctatgct gcatccactt tgcaatcagg ggtcccatct     180
   cggttcagtg gcagtggatc tgggacagat ttcactctca ccatcagcag cctacagcct     240
   gaagatgttg caacttatta ctgtcaaagg tataaccgtg caccgtatac ttttggccag     300
   gggaccaagg tggaaatcaa acgaactgtg gcggcgccat ctgtcttcat cttcccgcca     360
   tctgatgagc agttgaaatc tggaactgcc tctgttgtgt gcctgctgaa taacttctat     420
   cctcgagagg ccaaagtaca gtggaaggtg gataacgccc tccaatcggg taactcccag     480
   gagagtgtca cagagcagga cagcaaggac agcacctaca gcctcagcag caccctgacg     540
   ctgagcaaag cagattacga gaaacacaaa gtctacgcct gcgaagtcac ccatcagggc     600
   ctgagctcgc ccgtcacaaa gagcttcaac aggggagagt gttag                     645
<210> 7
   <211> 675
   <212> DNA
   <213> Artificial
<220>
   <223> Fd chain
<400> 7
   gaggtgcagc tggtggagtc tgggggaggc ttggtacagc ccggcaggtc cctgagactc      60
   tcctgtgcgg cctctggatt cacctttgat gattatgcca tgcactgggt ccggcaagct     120
   ccagggaagg gcctggaatg ggtctcagct atcacttgga atagtggtca catagactat     180
   gcggactctg tggagggccg attcaccatc tccagagaca acgccaagaa ctccctgtat     240
   ctgcaaatga acagtctgag agctgaggat acggccgtat attactgtgc gaaagtctcg     300
   taccttagca ccgcgtcctc ccttgactat tggggccaag gaaccctggt caccgtctcg     360
   agtgctagct tcaagggccc atcggtcttc cccctggcac cctcctccaa gagcacctct     420
   gggggcacag cggccctggg ctgcctggtc aaggactact tccccgaacc ggtgacggtg     480
   tcgtggaact caggcgccct gaccagcggc gtgcacacct tcccggctgt cctacagtcc     540
   tcaggactct actccctcag cagcgtggtg accgtgccct ccagcagctt gggcacccag     600
   acctacatct gcaacgtgaa tcacaagccc agcaacacca aggtggacaa gagagttgag     660
   cccaaatctt gttaa                                                      675
<210> 8
   <211> 284
   <212> DNA
   <213> Pichia angusta
<400> 8
   tacagagctt tatatcacct tactgaacgc tagagtagac ccaattcccg gctcacacca      60
   cccttacatg cagagctaac caataaggta attaattaac actatatagc tcgtggtgaa     120
   cactggcccg gagtagtcat acgtgtaggt ttttggcgtg atgaaaatca ggtggcgcac     180
   gacttttcgt aaagttcggg agggagtgct gcaaacggca tataaggacc agtttttctc     240
   gcacattatc aattgctctt tagtacaaag ataatataga aaca                      284
<210> 9
   <211> 1085
   <212> DNA
   <213> Artificial
<220>
   <223> GAP promoter G418 gene
<400> 9
   gctatacaga gctttatatc accttactga acgctagagt agacccaatt cccggctcac      60
   accaccctta catgcagagc taaccaataa ggtaattaat taacactata tagctcgtgg     120
   tgaacactgg cccggagtag tcatacgtgt aggtttttgg cgtgatgaaa atcaggtggc     180
   gcacgacttt tcgtaaagtt cgggagggag tgctgcaaac ggcatataag gaccagtttt     240
   tctcgcacat tatcaattgc tctttagtac aaagataata tagaaaccat atgattgaac     300
   aagatggatt gcacgcaggt tctccggccg cttgggtgga gaggctattc ggctatgact     360
   gggcacaaca gacaatcggc tgctctgatg ccgccgtgtt ccggctgtca gcgcaggggc     420
   gcccggttct ttttgtcaag accgacctgt ccggtgccct gaatgaactg caggacgagg     480
   cagcgcggct atcgtggctg gccacgacgg gcgttccttg cgcagctgtg ctcgacgttg     540
   tcactgaagc gggaagggac tggctgctat tgggcgaagt gccggggcag gatctcctgt     600
   catctcacct tgctcctgcc gagaaagtat ccatcatggc tgatgcaatg cggcggctgc     660
   atacgcttga tccggctacc tgcccattcg accaccaagc gaaacatcgc atcgagcgag     720
   cacgtactcg gatggaagcc ggtcttgtcg atcaggatga tctggacgaa gagcatcagg     780
   ggctcgcgcc agccgaactg ttcgccaggc tcaaggcgcg catgcccgac ggcgaggatc     840
   tcgtcgtgac ccatggcgat gcctgcttgc cgaatatcat ggtggaaaat ggccgctttt     900
   ctggattcat cgactgtggc cggctgggtg tggcggaccg ctatcaggac atagcgttgg     960
   ctacccgtga tattgctgaa gagcttggcg gcgaatgggc tgaccgcttc ctcgtgcttt    1020
   acggtatcgc cgctcccgat tcgcagcgca tcgccttcta tcgccttctt gacgagttct    1080
   tctga                                                                1085
<210> 10
   <211> 2055
   <212> DNA
   <213> Pichia angusta
<400> 10
   atgtccattt ccagagacga cgaccaccca agaccgaagc gcgcaaggtc tggcaagcct     60
   aagaaagaga cccctccggc caaacggagg gtccggacag ggtgtttgac gtgtcgcagg    120
   aaacacaaaa agtgcgacga aaaccggccc aagtgcgact tctgcaccgc aaaggggctc    180
   gaatgtgtct ggccagaaga agtcaagaag aacgtttttg tcaacaactc gttcaaggac    240
   ttttgtttca gccaaagcaa gcatcgcgac aggttccgca agcccacaga ctttgatctg    300
   tctagctccc tgtccccact agactaccat ctcggtccag atgatccgcg cacacgcaaa    360
   aaggcctact cgctcgacgt cgccgaccac tatctcagcg catttgatgg ctattcgcgc    420
   aaaaacgagt atctcgacgc acctctcaca acagcatacc ctctgctcga gacgtccgag    480
   tcgcccctgt cgggtctctt caacgctcca caggagctgc caagccagct gaccagaact    540
   ccgagctttg gggccagctc gctcagctac tttgacacgc tgcaaagcta tagcatgaac    600
   ccttcactgt catcgggctc aaacgataaa atctcgaccg catcctccgt ctcgtccgac    660
   atgtctcttg ccaaaaagga cgccgacttt gagcatcgtt actcgtttgc agagccgcag    720
   aaaaagagca aattcggcct cgactactac gacctgccaa acacgctcaa cgacctcatg    780
   tgtctcttca tcgctaccaa ggatagcacc tccggtagca aactgcccaa caacgagacc    840
   attttcgaga tcttcactga caagtctacc atggaggctc cttccatcga cgaccatgag    900
   atgatgctgt tgatggaaac gtacattgat caaattgctc atttcctcaa caagtccgac    960
   gagacccaat gtaacatttt tatgaccaaa atcccagagc tcgcacacag atttccctcc   1020
   ctaaagtacg ctatcctggg acttgcctcc cgacaccttg agaaagtcag atccgattat 1080
   tcgggcgaaa aaacgttgca gtactatacc tattctttgc aacagcttag catgtgtctg 1140
   aactccgaca aaaacactct cgaggtggtt gccacgtgtg tcctgctgtg ctattttgag 1200
   attctgagca caaatccgga ctcctggcat tcgagactcg agaactgcgg ctccatgctc 1260
   aaagtctgtc atatcaacgc acgctcagaa acagagctgg aaaaagcttt attttggagc 1320
   tttgccgcgc tagacatcgg ctgctgcgcc gttggagaca gaaagaccgt tgttcgtcct 1380
   gaggactggt ttggcaccga cgccccttcg agtccgaccg agactggcac tgcgttgcaa 1440
   tccatgttgt atctgtcatg ccagatcttc cagctggtca gctcgaacga tgaccagtct 1500
   gaattcgaca gctgctggaa gtcactttgg aagcagctgg gcgactggga acgcgcccga 1560
   tcgagcaaca tgcgcggctt ctgttttaaa agagacaact cgttccctga gactctgttc 1620
   agcagctcga cggccctttg tgccaaccaa ctgttccaca catgctgtat tctgatgctc 1680
   cagacaaagc cgcggctcgt caagctacat gagccagaaa aacaagaaag gacccccgac 1740
   acattattcc aaagtgccat caaaacggag caaggcgagt cggtctcgac cagcagtctg 1800
   atgtcgcggt ctcagatttg gcacgccaag cagatcatcg gtatcaacgt gtgcaacatg 1860
   aagagcgctt cgtcacggag tctcgggtgc cagatcttgt cgctccaggc gatctggatc 1920
   gccggaaagc tgatctcctc ggctcacgag cattcgatta ttctgcaact cttgcagaga 1980
   ctagaatcgt ggtcgagtat gtcgatggat tggagagaaa aacaactgac tgagttctgg 2040
   aaacgcgagt gctga 2055
<210> 11
   <211> 2667
   <212> DNA
   <213> Pichia pastoris
<400> 11
   atgagtaccg cagccccaat caaggaagaa agccaatttg cccatttgac cctaatgaac      60
   aaggatatac cttcgaacgc aaaacaggca aagtcgaaag tttcagcggc ccctgctaag     120
   acgggctcca aatctgctgg tggatctggc aacaacaacg ctgcacctgt gaaaaaaaga     180
   gtccgcacgg gctgtttgac ctgccgaaag aagcacaaga aatgtgacga gaacagaaac     240
   ccaaaatgtg acttttgcac tttgaaaggc ttggaatgtg tctggccaga gaacaataag     300
   aagaatatct tcgttaacaa ctccatgaag gatttcttag gcaagaaaac ggtggatgga     360
   gctgatagtc tcaatttggc cgtgaatctg caacaacagc agagttcaaa cacaattgcc     420
   aatcaatcgc tttcctcaat tggattggaa agttttggtt acggctctgg tatcaaaaac     480
   gagtttaact tccaagactt gataggttca aactctggca gttcagatcc gacattttca     540
   gtagacgctg acgaggccca aaaactcgac atttccaaca agaacagtcg taagagacag     600
   aaactaggtt tgctgccggt cagcaatgca acttcccatt tgaacggttt caatggaatg 660
   tccaatggaa agtcacactc tttctcttca ccgtctggga ctaatgacga tgaactaagt 720
   ggcttgatgt tcaactcacc aagcttcaac cccctcacag ttaacgattc taccaacaac 780
   agcaaccaca atataggttt gtctccgatg tcatgcttat tttctacagt tcaagaagca 840
   tctcaaaaaa agcatggaaa ttccagtaga cacttttcat acccatctgg gccggaggac 900
   ctttggttca atgagttcca aaaacaggcc ctcacagcca atggagaaaa tgctgtccaa 960
   cagggagatg atgcttctaa gaacaacaca gccattccta aggaccagtc ttcgaactca 1020
   tcgattttca gttcacgttc tagtgcagct tctagcaact caggagacga tattggaagg 1080
   atgggcccat tctccaaagg accagagatt gagttcaact acgattcttt tttggaatcg 1140
   ttgaaggcag agtcaccctc ttcttcaaag tacaatctgc cggaaacttt gaaagagtac 1200
   atgaccctta gttcgtctca tctgaatagt caacactccg acactttggc aaatggcact 1260
   aacggtaact attctagcac cgtttccaac aacttgagct taagtttgaa ctccttctct 1320
   ttctctgaca agttctcatt gagtccacca acaatcactg acgccgaaaa gttttcattg 1380
   atgagaaact tcattgacaa catctcgcca tggtttgaca cttttgacaa taccaaacag 1440
   tttggaacaa aaattccagt tctggccaaa aaatgttctt cattgtacta tgccattctg 1500
   gctatatctt ctcgtcaaag agaaaggata aagaaagagc acaatgaaaa aacattgcaa 1560
   tgctaccaat actcactaca acagctcatc cctactgttc aaagctcaaa taatattgag 1620
   tacattatca catgtattct cctgagtgtg ttccacatca tgtctagtga accttcaacc 1680
   cagagggaca tcattgtgtc attggcaaaa tacattcaag catgcaacat aaacggattt 1740
   acatctaatg acaaactgga aaagagtatt ttctggaact atgtcaattt ggatttggct 1800
   acttgtgcaa tcggtgaaga gtcaatggtc attcctttta gctactgggt taaagagaca 1860
   actgactaca agaccattca agatgtgaag ccatttttca ccaagaagac tagcacgaca 1920
   actgacgatg acttggacga tatgtatgcc atctacatgc tgtacattag tggtagaatc 1980
   attaacctgt tgaactgcag agatgcgaag ctcaattttg agcccaagtg ggagtttttg 2040
   tggaatgaac tcaatgaatg ggaattgaac aaacccttga cctttcaaag tattgttcag 2100
   ttcaaggcca atgacgaatc gcagggcgga tcaacttttc caactgttct attctccaac 2160
   tctcgaagct gttacagtaa ccagctgtat catatgagct acatcatctt agtgcagaat 2220
   aaaccacgat tatacaaaat cccctttact acagtttctg cttcaatgtc atctccatcg 2280
   gacaacaaag ctgggatgtc tgcttccagc acacctgctt cagaccacca cgcttctggt 2340
   gatcatttgt ctccaagaag tgtagagccc tctctttcga caacgttgag ccctccgcct 2400
   aatgcaaacg gtgcaggtaa caagttccgc tctacgctct ggcatgccaa gcagatctgt 2460
   gggatttcta tcaacaacaa ccacaacagc aatctagcag ccaaagtgaa ctcattgcaa   2520
   ccattgtggc acgctggaaa gctaattagt tccaagtctg aacatacaca gttgctgaaa   2580
   ctgttgaaca accttgagtg tgcaacaggc tggcctatga actggaaggg caaggagtta   2640
   attgactact ggaatgttga agaatag                                       2667
<210> 12
   <211> 2790
   <212> DNA
   <213> Pichia angusta
<400> 12
   atgtgtaagc gcaagagtac taacctggat gtgcccgaag cctcgtcgca aagctcatta      60
   cctgatcaag ggtcctccat cccagcaaac aaagaattag gccgagagat attaatgaag     120
   cccagcaact cggcgccgcc aagaatcatc atgccagcag gatcggacgg gtcgtcgggc     180
   gttgttttgc agaagaagcg ccgtgtggtg cgtgcctgcg acagatgtcg aaagctgaaa     240
   atcaagtgtt ctggcgacct gccctgcata cactgcactg tctacagcta cgagtgcacc     300
   tacgaccagc ccagtcgcgc aaagaggaag aacaccaccg ctaatgcagg gtctccatcg     360
   ggtccatctg ccaattctca gcatccccat cttcctacca acagcctgga ggtgatggcg     420
   cggatggccg accgactcaa gctgtacgac gacatcctgc atcgactact gcccgacatc     480
   aagctgacag atctcaacga caatccgaag ccgataaacc ccatgaagct gctcacggct     540
   atgaacaagc tgcgagaaaa gtcgtgcgac gaaacaaaat cctcgagcaa gaccatcgca     600
   gaggagtacg aaaggttgcc ggacgtgccc ctgccaacac tgcccagcgt gccgaagcct     660
   gccccttcca cgaccgcggt caagacccac gagtcaccgt cggaacgctc gcacttctgc     720
   agtccgcatg gtggcggccc gcaccaggga atagacggtt cgtacgagtc cagcatgggc     780
   aaggagatca agatcattct gccgtctcgg gaggttgcgc tgaggctgat caccaagact     840
   tgggaaaacg cgtgtgttct gttccgtttc taccatcgac ccgccttcat tgaggatctg     900
   aacgagctct acgacacaga ccccagccag tacaccaaca agcagcagcg gtttctgccg     960
   ctcgtgtact cagtcatggc ctgcggcgcc ctctttctta agagtgatga gcagaccaac    1020
   ggctcgaaga atggcgagcc gacccctgag aacgaggttt ttgaggacga aggatacagg    1080
   tacttcattg cagcgcggaa actcatcgat atcactgaca ccagagacac gtacggcatc    1140
   cagacaattg taatgttgat catatttttg cagtgttcag ccaggttgtc gacatgctat    1200
   gcatacatcg gcatagcgtt gcgggcagct ctgcgagaag gtctacacag aaagctcgac    1260
   tacccgttca atccaatcga gctggaaatc cgaaaaagat tgttctggac tatctacaag    1320
   atggacattt acgtcaacac catgctgggg cttccgcgga cgatcagcga ggatgacttc    1380
   gaccaggaca tgccaatcga gctcgacgac gagaacatca cggtggacgg ctatcgcttc    1440
   gaaaaacagg gcgaccgact gtcgagctcg ggcattgcca acgcacatac gagactgatc 1500
   ttcatcatga agaagatcgt cacaaaacta taccccatca aaccacgaaa gatagaaaac 1560
   ggccgtcgcc ccgaattgat gacccacgat attgtatacc aactggaaat cgacttacag 1620
   gactggatga atagtcttcc tctagagctg aagcctggtg tcgaaccttc aagccagtac 1680
   ctgaaagcca acagacttct tcatatctcc tatctgcacg tgaagattat cttatacagg 1740
   cctttcatcc actatctttc gtcggaccac gtgaataggc cgaagtcatc taatgatctc 1800
   aaatgcatcg agaaggccaa gaattgtatt aacgtggcca gaattgtggt gaaactggca 1860
   gaggacatga tcaacaagaa catgttgagt gggtcttatt ggttttcgat atacacgatt 1920
   tttttctccg tcgcatgcct ggtctactat gttcattttg caccgcccgt cacgtctagc 1980
   ggaaattttg atcccgagta catcgcaatc aagaaggacg cagaaagcgg aaagagagtt 2040
   ttggacattc tgaaggacag cagcatggcc gcgcgaagaa catacaacat tctcaatgcg 2100
   ctgtttgagg agctaaaccg caaaactgcc aatgccagca tcagccacga accgccaaac 2160
   tctcacttcc ttacgccaca agttggaaag tctcaattcg agcgtgataa ccacaaaaaa 2220
   gatcaaatgg ctattccact gccaccgcag aatatgagac cccccgagta cgtgcaacag 2280
   ccctttaacg gctactcaaa aatggaaaat tcagagatca acaacatcgt taatggtgtg 2340
   aatttcattg atggtgtgtc caccggcatt aaccttaatc ttccttcgaa ctcggaagtg 2400
   gaggacgtcc cgtcgtcaat atctcctcca gatcagaatt ccaaatacaa gtatgttcct 2460
   ggtcctatgg accaactgga catgaagatt ttcggacgct ttctgcctcc ttatatgctc 2520
   aattcaaatc aacagtcgac agataatact ccggagcccg agccttaccc agagcagtat 2580
   caagagccgg aaaacgagcc tcaaaatgga accatcttcg gatcaattcc ttcgggactg 2640
   tctaccgtct cccaatccca acaaaacagc aaaggtttta ccggactgcc gcccgactac 2700
   caaggcccac caaaacgtta cgattttgag gacatgttct ataatgactg gagtgcaggc 2760
   cctccaaatg attttaacca gaaatcataa 2790
<210> 13
   <211> 50
   <212> DNA
   <213> Artificial
<220>
   <223> Malti cloning site
<400> 13
   aagcttgcgg ccgcggatcc actagtcaat tgagatcttc tagagaattc 50
<210> 14
   <211> 35
   <212> DNA
   <213> Artificial
<220>
   <223> Primer1
<400> 14
   taaagcttga gtccctgagt acgtaagcgg tttgg 35
<210> 15
   <211> 33
   <212> DNA
   <213> Artificial
<220>
   <223> Primer2
<400> 15
   ataagcttat tatttaggaa ttattcgaga tcc 33
<210> 16
   <211> 30
   <212> DNA
   <213> Artificial
<220>
   <223> Primer3
<400> 16
   aatggatcct cgacgcggag aacgatctcc 30
<210> 17
   <211> 31
   <212> DNA
   <213> Artificial
<220>
   <223> Primer4
<400> 17
   attggatcct ttgtttttgt actttagatt g 31
<210> 18
   <211> 31
   <212> DNA
   <213> Artificial
<220>
   <223> Primer5
<400> 18
   ttagaattca acatccaaag acgaaaggtt g 31
<210> 19
   <211> 35
   <212> DNA
   <213> Artificial
<220>
   <223> Primer6
<400> 19
   aatgaattct tcgaataatt agttgttttt tgatc 35
<210> 20
   <211> 30
   <212> DNA
   <213> Artificial
<220>
   <223> Primer7
<400> 20
   aatcaattgt cgacgcggag aacgatctcc 30
<210> 21
   <211> 31
   <212> DNA
   <213> Artificial
<220>
   <223> Primer8
<400> 21
   attcaattgt ttgtttttgt actttagatt g 31
<210> 22
   <211> 31
   <212> DNA
   <213> Artificial
<220>
   <223> Primer9
<400> 22
   aatggatcca acatccaaag acgaaaggtt g 31
<210> 23
   <211> 35
   <212> DNA
   <213> Artificial
<220>
   <223> Primer10
<400> 23
   attggatcct tcgaataatt agttgttttt tgatc 35
<210> 24
   <211> 37
   <212> DNA
   <213> Artificial
<220>
   <223> Primer11
<400> 24
   taatctagag gagacgtgga aggacatacc gcttttg 37
<210> 25
   <211> 26
   <212> DNA
   <213> Artificial
<220>
   <223> Primer12
<400> 25
   gatcctctag acagtccgga agcgac 26
<210> 26
   <211> 32
   <212> DNA
   <213> Artificial
<220>
   <223> Primer13
<400> 26
   tccactagta tgagatttcc ttcaattttt ac 32
<210> 27
   <211> 24
   <212> DNA
   <213> Artificial
<220>
   <223> Primerl4
<400> 27
   tcttttctcg agagataccc cttc 24
<210> 28
   <211> 39
   <212> DNA
   <213> Artificial
<220>
   <223> Primerl5
<400> 28
   ggtatctctc gagaaaagag acatccagat gacccagtc 39
<210> 29
   <211> 32
   <212> DNA
   <213> Artificial
<220>
   <223> Primerl6
<400> 29
   tccactagtt taacactctc ccctgttgaa gc 32
<210> 30
   <211> 32
   <212> DNA
   <213> Artificial
<220>
   <223> Primerl7
<400> 30
   tccagatcta tgagatttcc ttcaattttt ac 32
<210> 31
   <211> 39
   <212> DNA
   <213> Artificial
<220>
   <223> Primerl8
<400> 31
   ggtatctctc gagaaaagag aggtgcagct ggtggagtc 39
<210> 32
   <211> 27
   <212> DNA
   <213> Artificial
<220>
   <223> Primerl9
<400> 32
   ttgagatctt taacaagatt tgggctc 27
<210> 33
   <211> 28
   <212> DNA
   <213> Artificial
<220>
   <223> Primer20
<400> 33
   ttagaattct acagagcttt atatcacc 28
<210> 34
   <211> 28
   <212> DNA
   <213> Artificial
<220>
   <223> Primer21
<400> 34
   aatgaattct gtttctatat tatctttg 28
<210> 35
   <211> 29
   <212> DNA
   <213> Artificial
<220>
   <223> Primer22
<400> 35
   aatgaattcc ccgctcagaa gaactcgtc 29
<210> 36
   <211> 28
   <212> DNA
   <213> Artificial
<220>
   <223> Primer23
<400> 36
   ttaggatcca tgtccatttc cagagacg 28
<210> 37
   <211> 28
   <212> DNA
   <213> Artificial
<220>
   <223> Primer24
<400> 37
   ttaggatcct cagcactcgc gtttccag 28
<210> 38
   <211> 684
   <212> PRT
   <213> Pichia angusta
<400> 38
   Met Ser Ile Ser Arg Asp Asp Asp His Pro Arg Pro Lys Arg Ala Arg
   1               5                   10                  15
   Ser Gly Lys Pro Lys Lys Glu Thr Pro Pro Ala Lys Arg Arg Val Arg
               20                  25                  30
   Thr Gly Cys Leu Thr Cys Arg Arg Lys His Lys Lys Cys Asp Glu Asn
           35                  40                  45
   Arg Pro Lys Cys Asp Phe Cys Thr Ala Lys Gly Leu Glu Cys Val Trp
       50                  55                  60
   Pro Glu Glu Val Lys Lys Asn Val Phe Val Asn Asn Ser Phe Lys Asp
   65                  70                  75                  80
   Phe Cys Phe Ser Gln Ser Lys His Arg Asp Arg Phe Arg Lys Pro Thr
                   85                  90                  95
   Asp Phe Asp Leu Ser Ser Ser Leu Ser Pro Leu Asp Tyr His Leu Gly
               100                 105                 110
   Pro Asp Asp Pro Arg Thr Arg Lys Lys Ala Tyr Ser Leu Asp Val Ala
           115                 120                 125
   Asp His Tyr Leu Ser Ala Phe Asp Gly Tyr Ser Arg Lys Asn Glu Tyr
       130                 135                 140
   Leu Asp Ala Pro Leu Thr Thr Ala Tyr Pro Leu Leu Glu Thr Ser Glu
   145                 150                 155                 160
   Ser Pro Leu Ser Gly Leu Phe Asn Ala Pro Gln Glu Leu Pro Ser Gln
                   165                 170                 175
   Leu Thr Arg Thr Pro Ser Phe Gly Ala Ser Ser Leu Ser Tyr Phe Asp
               180                 185                 190
   Thr Leu Gln Ser Tyr Ser Met Asn Pro Ser Leu Ser Ser Gly Ser Asn
           195                 200                 205
   Asp Lys Ile Ser Thr Ala Ser Ser Val Ser Ser Asp Met Ser Leu Ala
       210                 215                 220
   Lys Lys Asp Ala Asp Phe Glu His Arg Tyr Ser Phe Ala Glu Pro Gln
   225                 230                 235                 240
   Lys Lys Ser Lys Phe Gly Leu Asp Tyr Tyr Asp Leu Pro Asn Thr Leu
                   245                 250                 255
   Asn Asp Leu Met Cys Leu Phe Ile Ala Thr Lys Asp Ser Thr Ser Gly
               260                 265                 270
   Ser Lys Leu Pro Asn Asn Glu Thr Ile Phe Glu Ile Phe Thr Asp Lys
           275                 280                 285
   Ser Thr Met Glu Ala Pro Ser Ile Asp Asp His Glu Met Met Leu Leu
       290                 295                 300
   Met Glu Thr Tyr Ile Asp Gln Ile Ala His Phe Leu Asn Lys Ser Asp
   305                 310                 315                 320
   Glu Thr Gln Cys Asn Ile Phe Met Thr Lys Ile Pro Glu Leu Ala His
                   325                 330                 335
   Arg Phe Pro Ser Leu Lys Tyr Ala Ile Leu Gly Leu Ala Ser Arg His
               340                 345                 350
   Leu Glu Lys Val Arg Ser Asp Tyr Ser Gly Glu Lys Thr Leu Gln Tyr
           355                 360                 365
   Tyr Thr Tyr Ser Leu Gln Gln Leu Ser Met Cys Leu Asn Ser Asp Lys
       370                 375                 380
   Asn Thr Leu Glu Val Val Ala Thr Cys Val Leu Leu Cys Tyr Phe Glu
   385                 390                 395                 400
   Ile Leu Ser Thr Asn Pro Asp Ser Trp His Ser Arg Leu Glu Asn Cys
                   405                 410                 415
   Gly Ser Met Leu Lys Val Cys His Ile Asn Ala Arg Ser Glu Thr Glu
               420                 425                 430
   Leu Glu Lys Ala Leu Phe Trp Ser Phe Ala Ala Leu Asp Ile Gly Cys
           435                 440                 445
   Cys Ala Val Gly Asp Arg Lys Thr Val Val Arg Pro Glu Asp Trp Phe
       450                 455                 460
   Gly Thr Asp Ala Pro Ser Ser Pro Thr Glu Thr Gly Thr Ala Leu Gln
   465                 470                 475                 480
   Ser Met Leu Tyr Leu Ser Cys Gln Ile Phe Gln Leu Val Ser Ser Asn
                   485                 490                 495
   Asp Asp Gln Ser Glu Phe Asp Ser Cys Trp Lys Ser Leu Trp Lys Gln
               500                 505                 510
   Leu Gly Asp Trp Glu Arg Ala Arg Ser Ser Asn Met Arg Gly Phe Cys
           515                 520                 525
   Phe Lys Arg Asp Asn Ser Phe Pro Glu Thr Leu Phe Ser Ser Ser Thr
       530                 535                 540
   Ala Leu Cys Ala Asn Gln Leu Phe His Thr Cys Cys Ile Leu Met Leu
   545                 550                 555                 560
   Gln Thr Lys Pro Arg Leu Val Lys Leu His Glu Pro Glu Lys Gln Glu
                   565                 570                 575
   Arg Thr Pro Asp Thr Leu Phe Gln Ser Ala Ile Lys Thr Glu Gln Gly
               580                 585                 590
   Glu Ser Val Ser Thr Ser Ser Leu Met Ser Arg Ser Gln Ile Trp His
           595                 600                 605
   Ala Lys Gln Ile Ile Gly Ile Asn Val Cys Asn Met Lys Ser Ala Ser
   610 615 620
   Ser Arg Ser Leu Gly Cys Gln Ile Leu Ser Leu Gln Ala Ile Trp Ile
   625 630 635 640
   Ala Gly Lys Leu Ile Ser Ser Ala His Glu His Ser Ile Ile Leu Gln
   645 650 655
   Leu Leu Gln Arg Leu Glu Ser Trp Ser Ser Met Ser Met Asp Trp Arg
   660 665 670
   Glu Lys Gln Leu Thr Glu Phe Trp Lys Arg Glu Cys
   675 680
<210> 39
   <211> 30
   <212> DNA
   <213> Artificial
<220>
   <223> Primer25
<400> 39
   aatggatcca tgagtaccgc agccccaatc 30
<210> 40
   <211> 31
   <212> DNA
   <213> Artificial
<220>
   <223> Primer26
<400> 40
   attggatccc tattcttcaa cattccagta g 31
<210> 41
   <211> 888
   <212> PRT
   <213> Pichia pastoris
<400> 41
   Met Ser Thr Ala Ala Pro Ile Lys Glu Glu Ser Gln Phe Ala His Leu
   1 5 10 15
   Thr Leu Met Asn Lys Asp Ile Pro Ser Asn Ala Lys Gln Ala Lys Ser
   20 25 30
   Lys Val Ser Ala Ala Pro Ala Lys Thr Gly Ser Lys Ser Ala Gly Gly
   35 40 45
   Ser Gly Asn Asn Asn Ala Ala Pro Val Lys Lys Arg Val Arg Thr Gly
       50                  55                  60
   Cys Leu Thr Cys Arg Lys Lys His Lys Lys Cys Asp Glu Asn Arg Asn
   65                  70                  75                  80
   Pro Lys Cys Asp Phe Cys Thr Leu Lys Gly Leu Glu Cys Val Trp Pro
                   85                  90                  95
   Glu Asn Asn Lys Lys Asn Ile Phe Val Asn Asn Ser Met Lys Asp Phe
               100                 105                 110
   Leu Gly Lys Lys Thr Val Asp Gly Ala Asp Ser Leu Asn Leu Ala Val
           115                 120                 125
   Asn Leu Gln Gln Gln Gln Ser Ser Asn Thr Ile Ala Asn Gln Ser Leu
       130                 135                 140
   Ser Ser Ile Gly Leu Glu Ser Phe Gly Tyr Gly Ser Gly Ile Lys Asn
   145                 150                 155                 160
   Glu Phe Asn Phe Gln Asp Leu Ile Gly Ser Asn Ser Gly Ser Ser Asp
                   165                 170                 175
   Pro Thr Phe Ser Val Asp Ala Asp Glu Ala Gln Lys Leu Asp Ile Ser
               180                 185                 190
   Asn Lys Asn Ser Arg Lys Arg Gln Lys Leu Gly Leu Leu Pro Val Ser
           195                 200                 205
   Asn Ala Thr Ser His Leu Asn Gly Phe Asn Gly Met Ser Asn Gly Lys
       210                 215                 220
   Ser His Ser Phe Ser Ser Pro Ser Gly Thr Asn Asp Asp Glu Leu Ser
   225                 230                 235                 240
   Gly Leu Met Phe Asn Ser Pro Ser Phe Asn Pro Leu Thr Val Asn Asp
                   245                 250                 255
   Ser Thr Asn Asn Ser Asn His Asn Ile Gly Leu Ser Pro Met Ser Cys
               260                 265                 270
   Leu Phe Ser Thr Val Gln Glu Ala Ser Gln Lys Lys His Gly Asn Ser
           275                 280                 285
   Ser Arg His Phe Ser Tyr Pro Ser Gly Pro Glu Asp Leu Trp Phe Asn
   290 295 300
   Glu Phe Gln Lys Gln Ala Leu Thr Ala Asn Gly Glu Asn Ala Val Gln
   305 310 315 320
   Gln Gly Asp Asp Ala Ser Lys Asn Asn Thr Ala Ile Pro Lys Asp Gln
   325 330 335
   Ser Ser Asn Ser Ser Ile Phe Ser Ser Arg Ser Ser Ala Ala Ser Ser
   340 345 350
   Asn Ser Gly Asp Asp Ile Gly Arg Met Gly Pro Phe Ser Lys Gly Pro
   355 360 365
   Glu Ile Glu Phe Asn Tyr Asp Ser Phe Leu Glu Ser Leu Lys Ala Glu
   370 375 380
   Ser Pro Ser Ser Ser Lys Tyr Asn Leu Pro Glu Thr Leu Lys Glu Tyr
   385 390 395 400
   Met Thr Leu Ser Ser Ser His Leu Asn Ser Gln His Ser Asp Thr Leu
   405 410 415
   Ala Asn Gly Thr Asn Gly Asn Tyr Ser Ser Thr Val Ser Asn Asn Leu
   420 425 430
   Ser Leu Ser Leu Asn Ser Phe Ser Phe Ser Asp Lys Phe Ser Leu Ser
   435 440 445
   Pro Pro Thr Ile Thr Asp Ala Glu Lys Phe Ser Leu Met Arg Asn Phe
   450 455 460
   Ile Asp Asn Ile Ser Pro Trp Phe Asp Thr Phe Asp Asn Thr Lys Gln
   465 470 475 480
   Phe Gly Thr Lys Ile Pro Val Leu Ala Lys Lys Cys Ser Ser Leu Tyr
   485 490 495
   Tyr Ala Ile Leu Ala Ile Ser Ser Arg Gln Arg Glu Arg Ile Lys Lys
   500 505 510
   Glu His Asn Glu Lys Thr Leu Gln Cys Tyr Gln Tyr Ser Leu Gln Gln
   515 520 525
   Leu Ile Pro Thr Val Gln Ser Ser Asn Asn Ile Glu Tyr Ile Ile Thr
   530 535 540
   Cys Ile Leu Leu Ser Val Phe His Ile Met Ser Ser Glu Pro Ser Thr
   545                 550                 555                 560
   Gln Arg Asp Ile Ile Val Ser Leu Ala Lys Tyr Ile Gln Ala Cys Asn
                   565                 570                 575
   Ile Asn Gly Phe Thr Ser Asn Asp Lys Leu Glu Lys Ser Ile Phe Trp
               580                 585                 590
   Asn Tyr Val Asn Leu Asp Leu Ala Thr Cys Ala Ile Gly Glu Glu Ser
           595                 600                 605
   Met Val Ile Pro Phe Ser Tyr Trp Val Lys Glu Thr Thr Asp Tyr Lys
       610                 615                 620
   Thr Ile Gln Asp Val Lys Pro Phe Phe Thr Lys Lys Thr Ser Thr Thr
   625                 630                 635                 640
   Thr Asp Asp Asp Leu Asp Asp Met Tyr Ala Ile Tyr Met Leu Tyr Ile
                   645                 650                 655
   Ser Gly Arg Ile Ile Asn Leu Leu Asn Cys Arg Asp Ala Lys Leu Asn
               660                 665                 670
   Phe Glu Pro Lys Trp Glu Phe Leu Trp Asn Glu Leu Asn Glu Trp Glu
           675                 680                 685
   Leu Asn Lys Pro Leu Thr Phe Gln Ser Ile Val Gln Phe Lys Ala Asn
       690                 695                 700
   Asp Glu Ser Gln Gly Gly Ser Thr Phe Pro Thr Val Leu Phe Ser Asn
   705                 710                 715                 720
   Ser Arg Ser Cys Tyr Ser Asn Gln Leu Tyr His Met Ser Tyr Ile Ile
                   725                 730                 735
   Leu Val Gln Asn Lys Pro Arg Leu Tyr Lys Ile Pro Phe Thr Thr Val
               740                 745                 750
   Ser Ala Ser Met Ser Ser Pro Ser Asp Asn Lys Ala Gly Met Ser Ala
           755                 760                 765
   Ser Ser Thr Pro Ala Ser Asp His His Ala Ser Gly Asp His Leu Ser
       770                 775                 780
   Pro Arg Ser Val Glu Pro Ser Leu Ser Thr Thr Leu Ser Pro Pro Pro
   785                 790                 795                 800
   Asn Ala Asn Gly Ala Gly Asn Lys Phe Arg Ser Thr Leu Trp His Ala
                   805                 810                 815
   Lys Gln Ile Cys Gly Ile Ser Ile Asn Asn Asn His Asn Ser Asn Leu
               820                 825                 830
   Ala Ala Lys Val Asn Ser Leu Gln Pro Leu Trp His Ala Gly Lys Leu
           835                 840                 845
   Ile Ser Ser Lys Ser Glu His Thr Gln Leu Leu Lys Leu Leu Asn Asn
       850                 855                 860
   Leu Glu Cys Ala Thr Gly Trp Pro Met Asn Trp Lys Gly Lys Glu Leu
   865                 870                 875                 880
   Ile Asp Tyr Trp Asn Val Glu Glu
                   885
<210> 42
   <211> 29
   <212> DNA
   <213> Artificial
<220>
   <223> Primer27
<400> 42
   ttaactagta tgtgtaagcg caagagtac 29
<210> 43
   <211> 36
   <212> DNA
   <213> Artificial
<220>
   <223> Primer28
<400> 43
   ttaggatcct tatgatttct ggttaaaatc atttgg 36
<210> 44
   <211> 28
   <212> DNA
   <213> Artificial
<220>
   <223> Primer29
<400> 44
   ttaggatcct acagagcttt atatcacc 28
<210> 45
   <211> 28
   <212> DNA
   <213> Artificial
<220>
   <223> Primer30
<400> 45
   aatggatcct gtttctatat tatctttg 28
<210> 46
   <211> 929
   <212> PRT
   <213> Pichia angusta
<400> 46
   Met Cys Lys Arg Lys Ser Thr Asn Leu Asp Val Pro Glu Ala Ser Ser
   1               5                   10                  15
   Gln Ser Ser Leu Pro Asp Gln Gly Ser Ser Ile Pro Ala Asn Lys Glu
               20                  25                  30
   Leu Gly Arg Glu Ile Leu Met Lys Pro Ser Asn Ser Ala Pro Pro Arg
           35                  40                  45
   Ile Ile Met Pro Ala Gly Ser Asp Gly Ser Ser Gly Val Val Leu Gln
       50                  55                  60
   Lys Lys Arg Arg Val Val Arg Ala Cys Asp Arg Cys Arg Lys Leu Lys
   65                  70                  75                  80
   Ile Lys Cys Ser Gly Asp Leu Pro Cys Ile His Cys Thr Val Tyr Ser
                   85                  90                  95
   Tyr Glu Cys Thr Tyr Asp Gln Pro Ser Arg Ala Lys Arg Lys Asn Thr
               100                 105                 110
   Thr Ala Asn Ala Gly Ser Pro Ser Gly Pro Ser Ala Asn Ser Gln His
           115                 120                 125
   Pro His Leu Pro Thr Asn Ser Leu Glu Val Met Ala Arg Met Ala Asp
       130                 135                 140
   Arg Leu Lys Leu Tyr Asp Asp Ile Leu His Arg Leu Leu Pro Asp Ile
   145                 150                 155                 160
   Lys Leu Thr Asp Leu Asn Asp Asn Pro Lys Pro Ile Asn Pro Met Lys
                   165                 170                 175
   Leu Leu Thr Ala Met Asn Lys Leu Arg Glu Lys Ser Cys Asp Glu Thr
               180                 185                 190
   Lys Ser Ser Ser Lys Thr Ile Ala Glu Glu Tyr Glu Arg Leu Pro Asp
           195                 200                 205
   Val Pro Leu Pro Thr Leu Pro Ser Val Pro Lys Pro Ala Pro Ser Thr
       210                 215                 220
   Thr Ala Val Lys Thr His Glu Ser Pro Ser Glu Arg Ser His Phe Cys
   225                 230                 235                 240
   Ser Pro His Gly Gly Gly Pro His Gln Gly Ile Asp Gly Ser Tyr Glu
                   245                 250                 255
   Ser Ser Met Gly Lys Glu Ile Lys Ile Ile Leu Pro Ser Arg Glu Val
               260                 265                 270
   Ala Leu Arg Leu Ile Thr Lys Thr Trp Glu Asn Ala Cys Val Leu Phe
           275                 280                 285
   Arg Phe Tyr His Arg Pro Ala Phe Ile Glu Asp Leu Asn Glu Leu Tyr
       290                 295                 300
   Asp Thr Asp Pro Ser Gln Tyr Thr Asn Lys Gln Gln Arg Phe Leu Pro
   305                 310                 315                 320
   Leu Val Tyr Ser Val Met Ala Cys Gly Ala Leu Phe Leu Lys Ser Asp
                   325                 330                 335
   Glu Gln Thr Asn Gly Ser Lys Asn Gly Glu Pro Thr Pro Glu Asn Glu
               340                 345                 350
   Val Phe Glu Asp Glu Gly Tyr Arg Tyr Phe Ile Ala Ala Arg Lys Leu
           355                 360                 365
   Ile Asp Ile Thr Asp Thr Arg Asp Thr Tyr Gly Ile Gln Thr Ile Val
       370                 375                 380
   Met Leu Ile Ile Phe Leu Gln Cys Ser Ala Arg Leu Ser Thr Cys Tyr
   385                 390                 395                 400
   Ala Tyr Ile Gly Ile Ala Leu Arg Ala Ala Leu Arg Glu Gly Leu His
                   405                 410                 415
   Arg Lys Leu Asp Tyr Pro Phe Asn Pro Ile Glu Leu Glu Ile Arg Lys
               420                 425                 430
   Arg Leu Phe Trp Thr Ile Tyr Lys Met Asp Ile Tyr Val Asn Thr Met
   435 440 445
   Leu Gly Leu Pro Arg Thr Ile Ser Glu Asp Asp Phe Asp Gln Asp Met
   450 455 460
   Pro Ile Glu Leu Asp Asp Glu Asn Ile Thr Val Asp Gly Tyr Arg Phe
   465 470 475 480
   Glu Lys Gln Gly Asp Arg Leu Ser Ser Ser Gly Ile Ala Asn Ala His
   485 490 495
   Thr Arg Leu Ile Phe Ile Met Lys Lys Ile Val Thr Lys Leu Tyr Pro
   500 505 510
   Ile Lys Pro Arg Lys Ile Glu Asn Gly Arg Arg Pro Glu Leu Met Thr
   515 520 525
   His Asp Ile Val Tyr Gln Leu Glu Ile Asp Leu Gln Asp Trp Met Asn
   530 535 540
   Ser Leu Pro Leu Glu Leu Lys Pro Gly Val Glu Pro Ser Ser Gln Tyr
   545 550 555 560
   Leu Lys Ala Asn Arg Leu Leu His Ile Ser Tyr Leu His Val Lys Ile
   565 570 575
   Ile Leu Tyr Arg Pro Phe Ile His Tyr Leu Ser Ser Asp His Val Asn
   580 585 590
   Arg Pro Lys Ser Ser Asn Asp Leu Lys Cys Ile Glu Lys Ala Lys Asn
   595 600 605
   Cys Ile Asn Val Ala Arg Ile Val Val Lys Leu Ala Glu Asp Met Ile
   610 615 620
   Asn Lys Asn Met Leu Ser Gly Ser Tyr Trp Phe Ser Ile Tyr Thr Ile
   625 630 635 640
   Phe Phe Ser Val Ala Cys Leu Val Tyr Tyr Val His Phe Ala Pro Pro
   645 650 655
   Val Thr Ser Ser Gly Asn Phe Asp Pro Glu Tyr Ile Ala Ile Lys Lys
   660 665 670
   Asp Ala Glu Ser Gly Lys Arg Val Leu Asp Ile Leu Lys Asp Ser Ser
   675 680 685
   Met Ala Ala Arg Arg Thr Tyr Asn Ile Leu Asn Ala Leu Phe Glu Glu
       690                 695                 700
   Leu Asn Arg Lys Thr Ala Asn Ala Ser Ile Ser His Glu Pro Pro Asn
   705                 710                 715                 720
   Ser His Phe Leu Thr Pro Gln Val Gly Lys Ser Gln Phe Glu Arg Asp
                   725                 730                 735
   Asn His Lys Lys Asp Gln Met Ala Ile Pro Leu Pro Pro Gln Asn Met
               740                 745                 750
   Arg Pro Pro Glu Tyr Val Gln Gln Pro Phe Asn Gly Tyr Ser Lys Met
           755                 760                 765
   Glu Asn Ser Glu Ile Asn Asn Ile Val Asn Gly Val Asn Phe Ile Asp
       770                 775                 780
   Gly Val Ser Thr Gly Ile Asn Leu Asn Leu Pro Ser Asn Ser Glu Val
   785                 790                 795                 800
   Glu Asp Val Pro Ser Ser Ile Ser Pro Pro Asp Gln Asn Ser Lys Tyr
                   805                 810                 815
   Lys Tyr Val Pro Gly Pro Met Asp Gln Leu Asp Met Lys Ile Phe Gly
               820                 825                 830
   Arg Phe Leu Pro Pro Tyr Met Leu Asn Ser Asn Gln Gln Ser Thr Asp
           835                 840                 845
   Asn Thr Pro Glu Pro Glu Pro Tyr Pro Glu Gln Tyr Gln Glu Pro Glu
       850                 855                 860
   Asn Glu Pro Gln Asn Gly Thr Ile Phe Gly Ser Ile Pro Ser Gly Leu
   865                 870                 875                 880
   Ser Thr Val Ser Gln Ser Gln Gln Asn Ser Lys Gly Phe Thr Gly Leu
                   885                 890                 895
   Pro Pro Asp Tyr Gln Gly Pro Pro Lys Arg Tyr Asp Phe Glu Asp Met
               900                 905                 910
   Phe Tyr Asn Asp Trp Ser Ala Gly Pro Pro Asn Asp Phe Asn Gln Lys
           915                 920                 925
   Ser
<210> 47
   <211> 29
   <212> DNA
   <213> Artificial
<220>
   <223> Primer31
<400> 47
   ttaggatcca tgtgtaagcg caagagtac 29
<210> 48
   <211> 36
   <212> DNA
   <213> Artificial
<220>
   <223> Primer32
<400> 48
   ttaactagtt tatgatttct ggttaaaatc atttgg 36
<210> 49
   <211> 40
   <212> PRT
   <213> Pichia angusta
<400> 49
   Lys Arg Arg Val Arg Thr Gly Cys Leu Thr Cys Arg Arg Lys His Lys
   1               5                   10                  15
   Lys Cys Asp Glu Asn Arg Pro Lys Cys Asp Phe Cys Thr Ala Lys Gly
               20                  25                  30
   Leu Glu Cys Val Trp Pro Glu Glu
           35                  40
<210> 50
   <211> 41
   <212> PRT
   <213> Pichia pastoris
<400> 50
   Lys Lys Arg Val Arg Thr Gly Cys Leu Thr Cys Arg Lys Lys His Lys
   1               5                   10                  15
   Lys Cys Asp Glu Asn Arg Asn Pro Lys Cys Asp Phe Cys Thr Leu Lys
               20                  25                  30
   Gly Leu Glu Cys Val Trp Pro Glu Asn
           35                  40
<210> 51
   <211> 30
   <212> DNA
   <213> Artificial
<220>
   <223> Primer33
<400> 51
   ttagcggccg ctacagagct ttatatcacc 30
<210> 52
   <211> 31
   <212> DNA
   <213> Artificial
<220>
   <223> Primer34
<400> 52
   ttacggccgt tattcttcaa cattccagta g 31

## Description

### Technical Field

The present invention is intended to improve protein production using a yeast host. The present invention relates to a yeast in which a particular transcription factor gene is introduced and/or activated and a method for producing a protein using such yeast.

### Background Art

In recent years, hosts suitable for proteins of interest, such as animal cells (e.g., CHO), insects (e.g., silk worm), insect cells, animals (e.g., chickens and cows), and microorganisms (e.g., *E. coli* and yeast), have been used in order to produce proteins via genetic recombination. In particular, yeast cells can be cultured in large-scale, high-density culture systems in cost-effective media, and proteins can be produced at low cost. In addition, proteins can be secreted and expressed in a culture solution of yeast cells by using a signal peptide or the like, therefore, a process for purifying proteins is easy. Further, yeast cells are advantageous in that they are eukaryotic organisms and post-translational modifications such as glycosylation is possible.

When a yeast is used as a host, however, productivity varies significantly depending on target proteins, and products may occasionally be influenced by the type of carbon source utilized. Examples of such influence include saccharification caused by glucose and oxidation caused by methanol.

In order to overcome such problems, use of methanol-assimilating yeasts, such as yeasts of the genus *Pichia,* yeasts of the genus *Candida,* and yeasts of the genus *Torulopsis* as hosts has been examined, and production of the green fluorescent protein, human serum albumin, the hepatitis B surface antigen, the human epidermal growth factor, and hirudin has been reported (Non-Patent Document 1).

Van Zupten et al., BMC Genomics, 11:1 (2010) discloses that MPP1 and Mut3 are overexpressed in P.angusta which employ methanol as carbon source. Abdelmoula-Souissi et al, Protein Expression and Purification, 54:283-288 (2007) discloses that Mut3 may be used as positive regulator controlled by an inducible promoter in P.angusta. In order to produce a protein, a nucleotide sequence region (i.e., a promoter) is necessary for transcription of a gene encoding such protein in a site upstream of such gene. In addition to a RNA polymerase that reads the nucleotide sequence of the template DNA and synthesizes (transcribes) complementary RNA, a variety of transcription regulators are involved in promotion or suppression of transcription.

Methanol-assimilating yeasts begin to utilize methanol as a nutrient source, such utilization is mediated by an alcohol oxidase (AOX) or methanol oxidase (MOX), and the AOX or MOX gene is transcribed prior thereto. A nucleotide sequence region (i.e., a promoter) for transcription of the AOX or MOX gene is located in an upstream region thereof. For example, an MOX promoter is known to have two different transcription initiation points depending on the carbon source (Non-Patent Document 2). Non-Patent Document 2 also suggests that a plurality of factors are associated with the MOX promoter.

Since the AOX and MOX promoters for methanol-assimilating yeasts are potent promoters, such promoters are used for protein production. In order to achieve the high-level production of a target protein via a general means, many problems still need to be resolved.

### Prior Art Documents

### Non-Patent Documents

Non-Patent Document 1: Gellissen, G., Appl. Microbiol. Biotechnol., Dec. 2000; 54(6): 741-50
Non-Patent Document 2: V., Genu et al., Eur. J. Biochem., 270, 2467-2475, 2003

### Summary of the Invention

### Problem to be Solved by the Invention

It is an object of the present invention to improve the production efficiency of a target protein using yeasts, and especially methanol assimilating yeasts, by enhancing or activating the transcription or translation levels through, for example, promoter modification, high-level expression of transcription factors, and modification of hosts. More particularly, the present invention is intended to identify a factor that activates a promoter.

### Means for Solving the Problem

The present invention is defined by the claims.

### Effects of the Invention

The present invention provides a yeast for transformation in which a promoter-activating factor are expressed at high levels. With the use of the yeast for transformation according to the present invention, a target protein can be secreted and produced efficiently.

### Brief Description of the Drawings

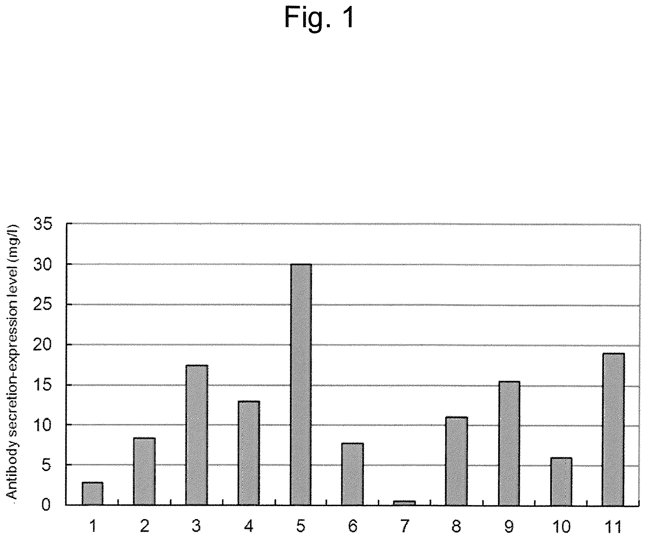

Fig. 1 shows the secretion-expression levels of a fully humanized anti-TNF-a Fab antibody by various transformed yeasts prepared in Example 8 (1: a transformant of *Pichia angusta* expressing a fully humanized anti-TNF-α Fab antibody; 2: a transformant of *Pichia angusta* expressing PaMPP1 (pUCPgapPaMPP1TmG418-introduced) and fully humanized anti-TNF-α Fab antibody; 3: a transformant of *Pichia angusta* expressing PpMPP1 (pUCPgapPpMPP1TmG418-introduced) and fully humanized anti-TNF-α Fab antibody; 4: a transformant of *Pichia angusta* expressing Mut3 (pUCPgapMut3TmG418-introduced) and fully humanized anti-TNF-α Fab antibody; 5: a transformant of *Pichia angusta* co-expressing PaMPP1 and Mut3 (pUCPgapMut3PgapPaMPP1TmG418-introduced) and fully humanized anti-TNF-α Fab antibody; 6: a transformant of *Pichia angusta* co-expressing PaMPP1, Mut3, and PpMPP1 (pUCPgapPpMPP1PgapMut3PgapPaMPP1TmG418-introduced) and fully humanized anti-TNF-α Fab antibody; 7: a transformant of *Pichia pastoris* expressing fully humanized anti-TNF-α Fab antibody; 8: a transformant of *Pichia pastoris* expressing PaMPP1 (pUCPgapPaMPP1TmG418-introduced) and fully humanized anti-TNF-α Fab antibody; 9: a transformant of *Pichia pastoris* expressing PpMPP1 (pUCPgapPpMPP1TmG418-introduced) and fully humanized anti-TNF-α Fab antibody; 10: a transformant of *Pichia pastoris* expressing Mut3 (pUCPgapMut3TmG418-introduced) and fully humanized anti-TNF-α Fab antibody; and 11: a transformant of *Pichia pastoris* co-expressing PaMPP1/Mut3 (pUCPgapMut3PgapPaMPP1TmG418-introduced) and fully humanized anti-TNF-α Fab antibody).

### Embodiments for Carrying out the Invention

Hereafter, the present invention is described in detail with reference to the preferred embodiments.

### 1. Yeast for transformation

The yeast for transformation of the present invention exhibits improved capacity to produce a target protein through the high-level expression of a factor that activates a promoter for transcription and expression of a gene encoding a target protein in the yeast. In the present invention, the term "factor that activates a promoter" refers to "MPP1" or a functional homolog thereof (hereafter, MPP1 and a functional homolog thereof are collectively referred to as "MPP1 homologs").

The term "promoter" refers to a nucleotide sequence region located upstream of a gene encoding a protein. In addition to RNA polymerase, a variety of transcription regulators involved in promotion and suppression of transcription bind to or act on such region to read the nucleotide sequence of the template gene and then synthesize (transcribe) complementary RNA.

"MPP1" is a protein involved in the regulation of peroxisome protein levels. Specifically, MPP1 is PaMPP1 as shown in SEQ ID NO: 38 derived from *Pichia angusta* described in "Transcriptional downregulation of peroxisome numbers affects selective peroxisome degradation in Hansenula polymorpha" (J. Biol. Chem., 2003, 278: 40749-40756) or PpMPP1 as shown in SEQ ID NO: 41 derived from *Pichia pastoris* with the GenBank Accession Number XP_002493066.

A functional homolog of MPP1 is a polypeptide having functions as a factor that activates a promoter equivalent to those of PaMPP1 or PpMPP1 derived from a yeast of the genus *Pichia* described above. Functional homologs of MPP1 may be isolated from *Eumycetes,* including other yeasts or molds. Examples include polypeptides, such as CBF78897 TPA: Putative Zn(II)2Cys6 transcription factor (Eurofung) (*Aspergillus nidulans* FGSC A4), CBF82226 TPA: Putative Zn(II)2Cys6 transcription factor (Eurofung) (*Aspergillus nidulans* FGSC A4), EDK37118 hypothetical protein PGUG_01216 (*Pichia guilliermondii* ATCC 6260), EDK41734 hypothetical protein PGUG_05832 (*Pichia guilliermondii* ATCC 6260), EDP47334 C6 transcription factor, putative (*Aspergillus fumigatus* A1163), EDP53358 C6 transcription factor, putative (*Aspergillus fumigatus* A1163), EEH16200 conserved hypothetical protein (*Paracoccidioides brasiliensis* Pb03), EEH22621 conserved hypothetical protein (*Paracoccidioides brasiliensis* Pb03), EEH42856 conserved hypothetical protein (*Paracoccidioides brasiliensis* Pb18), EEH49391 C6 zinc finger domain-containing protein (*Paracoccidioides brasiliensis* Pb18), EEQ42087 conserved hypothetical protein (*Candida albicans* WO-1), EEQ88974 C6 zinc finger domain-containing protein (*Ajellomyces dermatitidis* ER-3), EER38331 C6 zinc finger protein (*Ajellomyces capsulatus* H143), EFQ30548 hypothetical protein GLRG_05692 (*Glomerella graminicola* M1.001), EFQ34763 hypothetical protein GLRG_09907 (*Glomerella graminicola* M1.001), EFQ35786 C6 zinc finger domain-containing protein (*Glomerella graminicola* M1.001), EFQ86342 hypothetical protein PTT_18442 (*Pyrenophora teres f. teres* 0-1), EFQ86946 hypothetical protein PTT_17743 (*Pyrenophora teres f teres* 0-1), EFR00222 C6 zinc finger domain-containing protein (*Arthroderma gypseum* CBS 118893), XP_001210705 conserved hypothetical protein (*Aspergillus terreus* NIH2624), XP_001210874 conserved hypothetical protein (*Aspergillus terreus* NIH2624), XP_001212782 conserved hypothetical protein (*Aspergillus terreus* NIH2624), XP_001215119 conserved hypothetical protein (*Aspergillus terreus* NIH2624), XP_001216311 predicted protein (*Aspergillus terreus* NIH2624), XP_001216865 predicted protein (*Aspergillus terreus* NIH2624), XP_001216868 conserved hypothetical protein (*Aspergillus terreus* NIH2624), XP_001221354 hypothetical protein CHGG_02133 (*Chaetomium globosum* CBS 148.51), XP_001224209 hypothetical protein CHGG_04995 (*Chaetomium globosum* CBS 148.51), XP_001246901 hypothetical protein CIMG_00672 (*Coccidioides immitis* RS), XP_001257329 hypothetical protein NFIA_047680 (*Neosartorya fischeri* NRRL 181), XP_001257817 sexual development transcription factor RosA (*Neosartorya fischeri* NRRL 181), XP_001259009 C6 zinc finger domain protein (*Neosartorya fischeri* NRRL 181), XP_001259056 hypothetical protein NFIA_005210 (*Neosartorya fischeri* NRRL 181), XP_001259127 hypothetical protein NFIA_005940 (*Neosartorya fischeri* NRRL 181), XP_001259586 C6 zinc finger domain protein (*Neosartorya fischeri* NRRL 181), XP_001262439 hypothetical protein NFIA_029740 (*Neosartorya fischeri* NRRL 181), XP_001266583 hypothetical protein NFIA_101700 (*Neosartorya fischeri* NRRL 181), XP_001270410 conserved hypothetical protein (*Aspergillus clavatus* NRRL 1), XP_001270412 conserved hypothetical protein (*Aspergillus clavatus* NRRL 1), XP_001387003 predicted protein (*Scheffersomyces stipitis* CBS 6054), XP_001387109 predicted protein (*Scheffersomyces stipitis* CBS 6054), XP_001388653 hypothetical protein An01g02150 (*Aspergillus niger*), XP_001391257 hypothetical protein An07g01700 (*Aspergillus niger*), XP_001394851 hypothetical protein An11g08910 (*Aspergillus niger*), XP_001397318 hypothetical protein An16g00040 *(Aspergillus niger),* XP_001398002 hypothetical protein An16g07020 (*Aspergillus niger*), XP_001400256 hypothetical protein An02g11270 (*Aspergillus niger*), XP_001402162 hypothetical protein An04g08040 (*Aspergillus niger*), XP_001482069 hypothetical protein PGUG_05832 (*Meyerozyma guilliermondii* ATCC 6260), XP_001485545 hypothetical protein PGUG_01216 (*Meyerozyma guilliermondii* ATCC 6260), XP_001525560 conserved hypothetical protein (*Lodderomyces elongisporus* NRRL YB-4239), XP_001552259 hypothetical protein BC1G_08737 (*Botryotinia fuckeliana* B05.10), XP_001590624 hypothetical protein SS1G_08364 (*Sclerotinia sclerotiorum* 1980), XP_001791576 hypothetical protein SNOG_00909 (*Phaeosphaeria nodorum* SN15), XP_001798983 hypothetical protein SNOG_08674 (*Phaeosphaeria nodorum* SN15), XP_001800139 hypothetical protein SNOG_09853 (*Phaeosphaeria nodorum* SN15), XP_001800140 hypothetical protein SNOG_09854 (*Phaeosphaeria nodorum* SN15), XP_001820352 hypothetical protein (*Aspergillus oryzae* RIB40), XP_001820847 hypothetical protein (*Aspergillus oryzae* RIB40), XP_001822149 hypothetical protein (*Aspergillus oryzae* RIB40), XP_001825067 hypothetical protein (*Aspergillus oryzae* RIB40), XP_001827368 hypothetical protein (*Aspergillus oryzae* RIB40), XP_001933596 conserved hypothetical protein (*Pyrenophora tritici-repentis* Pt-1C-BFP), XP_001939276 C6 zinc finger domain containing protein (*Pyrenophora tritici-repentis* Pt-1C-BFP), XP_002145025 C6 transcription factor (ArcA), putative *(Penicillium marneffei* ATCC 18224), XP_002149655 conserved hypothetical protein (*Penicillium marneffei* ATCC 18224), XP_002149656 C6 transcription factor, putative (*Penicillium marneffei* ATCC 18224), XP_002152992 conserved hypothetical protein (*Penicillium marneffei* ATCC 18224), XP_002340358 C6 transcription factor (ArcA), putative (*Talaromyces stipitatus* ATCC 10500), XP_002374058 conserved hypothetical protein (*Aspergillus flavus* NRRL3357), XP_002375724 C6 transcription factor, putative (*Aspergillus flavus* NRRL3357), XP_002376570 conserved hypothetical protein (*Aspergillus flavus* NRRL3357), XP_002379231 C6 transcription factor (ArcA), putative (*Aspergillus flavus* NRRL3357), XP_002381760 C6 transcription factor, putative (*Aspergillus flavus* NRRL3357), XP_002417371 conserved hypothetical protein (*Candida dubliniensis* CD36), XP_002417723 zinc cluster transcription factor, putative *(Candida dubliniensis* CD36), XP_002481660 C6 transcription factor, putative (*Talaromyces stipitatus* ATCC 10500), XP_002484214 conserved hypothetical protein (*Talaromyces stipitatus* ATCC 10500), XP_002494969 ZYRO0B00242p (*Zygosaccharomyces rouxii*), XP_002498535 ZYRO0G12584p (*Zygosaccharomyces rouxii*), XP_002541155 predicted protein (*Uncinocarpus reesii* 1704), XP_002549474 hypothetical protein CTRG_03771 (*Candida tropicalis* MYA-3404), XP_002549678 predicted protein (*Candida tropicalis* MYA-3404), XP_002557168 Pc12g02800 (*Penicillium chrysogenum* Wisconsin 54-1255), XP_002557176 Pc12g02890 (*Penicillium chrysogenum* Wisconsin 54-1255), XP_002558943 Pc13g05080 (*Penicillium chrysogenum* Wisconsin 54-1255), XP_002560015 Pc14g00200 (*Penicillium chrysogenum* Wisconsin 54-1255), XP_002563281 Pc20g07580 (*Penicillium chrysogenum* Wisconsin 54-1255), XP_002567816 Pc21g07760 (*Penicillium chrysogenum* Wisconsin 54-1255), XP_002628177 C6 zinc finger domain-containing protein (*Ajellomyces dermatitidis* SLH14081), XP_002795625 conserved hypothetical protein (*Paracoccidioides brasiliensis* Pb01), XP_002848121 C6 zinc finger domain-containing protein (*Arthroderma otae* CBS 113480), XP_003001329 C6 zinc finger domain-containing protein (*Verticillium albo-atrum* VaMs.102), XP_003001370 C6 zinc finger domain-containing protein (*Verticillium albo-atrum* VaMs.102), XP_003002986 conserved hypothetical protein (*Verticillium albo-atrum* VaMs.102), XP_003002987 C6 zinc finger domain-containing protein (*Verticillium albo-atrum* VaMs.102), XP_003009478 C6 zinc finger domain-containing protein (*Verticillium albo-atrum* VaMs.102), XP_003010314 conserved hypothetical protein (*Arthroderma benhamiae* CBS 112371), XP_003024130 conserved hypothetical protein (*Trichophyton verrucosum* HKI 0517), XP_003040390 hypothetical protein NECHADRAFT_44605 (*Nectria haematococca* mpVI 77-13-4), XP_003040613 hypothetical protein NECHADRAFT_79789 (*Nectria haematococca* mpVI 77-13-4), XP_003042194 hypothetical protein NECHADRAFT_42482 (*Nectria haematococca* mpVI 77-13-4), XP_003045088 hypothetical protein NECHADRAFT_43849 (*Nectria haematococca* mpVI 77-13-4), XP_003051420 hypothetical protein NECHADRAFT_80852 (*Nectria haematococca* mpVI 77-13-4), XP_003051651 hypothetical protein NECHADRAFT_92631 (*Nectria haematococca* mpVI 77-13-4), XP_003052094 hypothetical protein NECHADRAFT_78932 (*Nectria haematococca* mpVI 77-13-4), XP_003068062 Fungal Zn binuclear cluster domain containing protein (*Coccidioides posadasii* C735 delta SOWgp), XP_363960 hypothetical protein MGG_01887 (*Magnaporthe oryzae* 70-15), XP_370281 hypothetical protein MGG_06778 (*Magnaporthe oryzae* 70-15), XP_384237 hypothetical protein FG04061.1 (*Gibberella zeae* PH-1), XP_384367 hypothetical protein FG04191.1 (*Gibberella zeae* PH-1), XP_388176 hypothetical protein FG08000.1 (*Gibberella zeae* PH-1), XP_391514 hypothetical protein FG11338.1 (*Gibberella zeae* PH-1), XP_456995 DEHA2B00638p (*Debaryomyces hansenii* CBS767), XP_460402 DEHA2F00946p (*Debaryomyces hansenii* CBS767), XP_570940 hypothetical protein CNE02410 (*Cryptococcus neoformans var. neoformans* JEC21), XP_659433 hypothetical protein AN1829.2 (*Aspergillus nidulans* FGSC A4), XP_660561 hypothetical protein AN2957.2 *(Aspergillus nidulans* FGSC A4), XP_662863 hypothetical protein AN5259.2 (*Aspergillus nidulans* FGSC A4), XP_664794 hypothetical protein AN7190.2 (*Aspergillus nidulans* FGSC A4), XP_746554 C6 transcription factor (*Aspergillus fumigatus* Af293), XP_748614 C6 transcription factor (*Aspergillus fumigatus* Af293), XP_751365 C6 transcription factor (*Aspergillus fumigatus* Af293), XP_753541 C6 transcription factor (ArcA) (*Aspergillus fumigatus* Af293), and XP_775527 hypothetical protein CNBE2410 (*Cryptococcus neoformans var. neoformans* B-3501A).

The MPP1 homolog of the present invention is preferably PaMPP1 or PpMPP1. Outside the scope of the invention are disclosed the following MPP1 homologs: CBF82226 TPA: Putative Zn(II)2Cys6 transcription factor (Eurofung) (*Aspergillus nidulans* FGSC A4), EDK37118 hypothetical protein PGUG_01216 (*Pichia guilliermondii* ATCC 6260), EDP47334 C6 transcription factor, putative (*Aspergillus fumigatus* A1163), EEH22621 conserved hypothetical protein (*Paracoccidioides brasiliensis* Pb03), EEH49391 C6 zinc finger domain-containing protein (*Paracoccidioides brasiliensis* Pbl8), EEQ42087 conserved hypothetical protein (*Candida albicans* WO-1), EFQ34763 hypothetical protein GLRG_09907 (*Glomerella graminicola* M1.001), EFQ86342 hypothetical protein PTT_18442 (*Pyrenophora teres f teres* 0-1), EFQ86946 hypothetical protein PTT_17743 (*Pyrenophora teres f. teres* 0-1), EFR00222 C6 zinc finger domain-containing protein (*Arthroderma gypseum* CBS 118893), XP_001216865 predicted protein (*Aspergillus terreus* NIH2624), XP_001246901 hypothetical protein CIMG_00672 (*Coccidioides immitis* RS), XP_001259586 C6 zinc finger domain protein (*Neosartorya fischeri* NRRL 181), XP_001262439 hypothetical protein NFIA_029740 (*Neosartorya fischeri* NRRL 181), XP_001387003 predicted protein (*Scheffersomyces stipitis* CBS 6054), XP_001485545 hypothetical protein PGUG_01216 (*Meyerozyma guilliermondii* ATCC 6260), XP_001525560 conserved hypothetical protein (*Lodderomyces elongisporus* NRRL YB-4239), XP_001552259 hypothetical protein BC1G_08737 (*Botryotinia fuckeliana* B05.10), XP_001798983 hypothetical protein SNOG_08674 (*Phaeosphaeria nodorum* SN15), XP_001933596 conserved hypothetical protein (*Pyrenophora tritici-repentis* Pt-1C-BFP), XP_001939276 C6 zinc finger domain containing protein (*Pyrenophora tritici-repentis* Pt-1C-BFP), XP_002152992 conserved hypothetical protein (*Penicillium marneffei* ATCC 18224), XP_002340358 C6 transcription factor (ArcA), putative (*Talaromyces stipitatus* ATCC 10500), XP_002379231 C6 transcription factor (ArcA), putative (*Aspergillus flavus* NRRL3357), XP_002417371 conserved hypothetical protein (*Candida dubliniensis* CD36), XP_002417723 zinc cluster transcription factor, putative (*Candida dubliniensis* CD36), XP_002481660 C6 transcription factor, putative (*Talaromyces stipitatus* ATCC 10500), XP_002494969 ZYRO0B00242p (*Zygosaccharomyces rouxii*), XP_002541155 predicted protein (*Uncinocarpus reesii* 1704), XP_002549474 hypothetical protein CTRG_03771 (*Candida tropicalis* MYA-3404), XP_002549678 predicted protein (*Candida tropicalis* MYA-3404), XP_002795625 conserved hypothetical protein (*Paracoccidioides brasiliensis* Pb01), XP_003002986 conserved hypothetical protein (*Verticillium albo-atrum* VaMs.102), XP_003009478 C6 zinc finger domain-containing protein (*Verticillium albo-atrum* VaMs.102), XP_003045088 hypothetical protein NECHADRAFT_43849 (*Nectria haematococca* mpVI 77-13-4), XP_003068062 Fungal Zn binuclear cluster domain containing protein (*Coccidioides posadasii* C735 delta SOWgp), XP_363960 hypothetical protein MGG_01887 (*Magnaporthe oryzae* 70-15), XP_384367 hypothetical protein FG04191.1 (*Gibberella zeae* PH-1), XP_456995 DEHA2B00638p (*Debaryomyces hansenii* CBS767), XP_746554 C6 transcription factor (*Aspergillus fumigatus* Af293), or XP_753541 C6 transcription factor (ArcA) (*Aspergillus fumigatus* Af293).

The MPP1 homolog of the present invention may be a polypeptide having an amino acid sequence having at least 85% or higher, preferably 90% or higher, more preferably 95% or higher, further preferably 97% or higher, and most preferably 99% or higher sequence identity with the amino acid sequence of the PaMPP1 or PpMPP1 homolog, provided that it is capable of exerting functions substantially equivalent to those of the MPP1 homolog.

A promoter activated by an MPP1 homolog is not particularly limited, provided that it is activated by an MPP1 homolog and capable of realizing high-level expression of the target protein in a yeast. A promoter activated by an MPP1 homolog is preferably a methanol-inducible promoter.

The methanol-inducible promoter of the present invention is not particularly limited, provided that it has transcription activity when a carbon source is methanol. For example, it is preferably the ADR1 promoter, the ALD promoter, the Alg3 promoter, the AMO promoter, the AOX1 promoter, the AOX2 promoter, the Atg1 promoter, the Atg2 promoter, the Atg3 promoter, the Atg4 promoter, the Atg5 promoter, the Atg6 promoter, the Atg7 promoter, the Atg8 promoter, the Atg9 promoter, the Atg10 promoter, the Atg11 promoter, the Atg12 promoter, the Atg13 promoter, the Atg15 promoter, the Atg16 promoter, the Atg17 promoter, the Atg18 promoter, the Atg19 promoter, the Atg21 promoter, the Atg22 promoter, the Atg24 promoter, the Atg26 promoter, the Atg28 promoter, the CAT promoter, the CNX promoter, the CPY promoter, the DAK promoter, the DAO promoter, the DHAS promoter, the DIC1 promoter, the DNM1 promoter, the FAO promoter, the FDH promoter, the FGH promoter, the FIG4 promoter, the FMD promoter, the Fmp22 promoter, the GAP promoter, the Gaslp promoter, the GCR1 promoter, the GDH promoter, the GSH promoter, the HARO7 promoter, the HpPmp20 promoter, the HSA1 promoter, the HSV2 promoter, the KEX1 promoter, the KEX2 promoter, the MALI promoter, the MAS promoter, the MDV1 promoter, the MLN promoter, the Mnn9p promoter, the MOX promoter, the MPP1 promoter, the Mut3 promoter, the Mxr1 promoter, the Nii2p promoter, the OCH promoter, the PAH2 promoter, the PAK1 promoter, the PDD1 promoter, the PEP4 promoter, the PEP12 promoter, the PepN promoter, the PER6 promoter, the PER10 promoter, the Pex1 promoter, the Pex3 promoter, the Pex4 promoter, the Pex5 promoter, the Pex6 promoter, the Pex7 promoter, the Pex8 promoter, the Pex10 promoter, the Pex11 promoter, the Pex12 promoter, the Pex13 promoter, the Pex17 promoter, the Pex19 promoter, the Pex20 promoter, the Pex22 promoter, the Pex23 promoter, the Pex24 promoter, the Pex25 promoter, the Pex26 promoter, the Pex32 promoter, the PHO1 promoter, the PIP2 promoter, the POX1 promoter, the PPI promoter, the PRAI promoter, the Prm1 promoter, the PYC promoter, the RHO promoter, the SLA2 promoter, the SNF1 promoter, the STL1 promoter, the Swilp promoter, the TOR promoter, the TPS1 promoter, the TPS3 promoter, the Trm1 promoter, the Trm2 promoter, the Vac8p promoter, the Vam3p promoter, the Vam7p promoter, the YAP promoter, the VPS1 promoter, the Yhflp promoter, the YNA1 promoter, the YNA2 promoter, the YNI1 promoter, the YNR1 promoter, the YNT1 promoter, the YPT1 promoter, or the Ypt7p promoter, and more preferably the AOX promoter, the DHAS promoter, the FMD promoter, or the MOX promoter.

The MPP1 homolog of the present invention is preferably PaMPP1, and PaMPP1 is not particularly limited, provided that it is a polypeptide having the function of a factor that activates the promoter mentioned above. In addition to a polypeptide consisting of the amino acid sequence as shown in SEQ ID NO: 38, it may be a polypeptide consisting of an amino acid sequence derived from the amino acid sequence as shown in SEQ ID NO: 38 by substitution, deletion, insertion, and/or addition of one or a plurality of amino acids or an amino acid sequence having 85% or higher, 90% or higher, 95% or higher, and more preferably 99% or higher sequence identity with the amino acid sequence as shown in SEQ ID NO: 38. The number of amino acids defined by the term "a plurality of amino acids" is not particularly limited, provided that functions of the MPP1 homolog are retained. The number of amino acids is up to 102, more preferably up to 100, further preferably up to 90, and most preferably up to 80, 70, 60, 50, 40, 30, 20, 10, 5, 4, 3, or 2.

The MPP1 homolog of the present invention is preferably PpMPP1, and PpMPP1 is not particularly limited, provided that it is a polypeptide having the function of factor that activates the promoter mentioned above. In addition to a polypeptide consisting of the amino acid sequence as shown in SEQ ID NO: 41, it may be a polypeptide consisting of an amino acid sequence derived from the amino acid sequence as shown in SEQ ID NO: 41 by substitution, deletion, insertion, and/or addition of one or a plurality of amino acids or an amino acid sequence having 85% or higher, 90% or higher, 95% or higher, and more preferably 99% or higher sequence identity with the amino acid sequence as shown in SEQ ID NO: 41. The number of amino acids defined by the term "a plurality of amino acids" is not particularly limited, provided that the functions of the MPP1 homolog are retained. The number of amino acids is up to 133, more preferably up to 130, further preferably up to 120, and most preferably up to 110, 100, 90, 80, 70, 60, 50, 40, 30, 20, 10, 5, 4, 3, or 2.

According to the present invention, amino acid sequence identity can be determined by a method well known in the art using sequence analysis software and the like. An example is a homology search conducted with the use of genetic information processing software (GENETYX Ver. 10, network edition, Genetyx).

The MPP1 homolog of the present invention may be a polypeptide consisting of the amino acid sequence as shown in SEQ ID NO: 49. PaMPP1 as shown in SEQ ID NO: 49 is a polypeptide composed of amino acid residues 28 to 67, which may be capable of binding to a promoter. By combining the polypeptide as shown in SEQ ID NO: 49 with another polypeptide and allowing expression of the protein in a host at high levels, the effects of promoter activation can be attained. In addition to the amino acid sequence as shown in SEQ ID NO: 49, it may be a polypeptide consisting of an amino acid sequence derived from the amino acid sequence as shown in SEQ ID NO: 49 by substitution, deletion, insertion, and/or addition of one or a plurality of amino acids or an amino acid sequence having 85% or higher, 90% or higher, 95% or higher, and more preferably 99% or higher sequence identity with the amino acid sequence as shown in SEQ ID NO: 49. The number of amino acids defined by the term "a plurality of amino acids" is not particularly limited, provided that functions of the MPP1 homolog are retained. The number of amino acids is preferably up to 6, more preferably up to 5, further preferably up to 4, and most preferably up to 3 or 2.

The MPP1 homolog of the present invention may be a polypeptide consisting of the amino acid sequence as shown in SEQ ID NO: 50. PpMPP1 as shown in SEQ ID NO: 50 is a polypeptide composed of amino acid residues 58 to 98, which may be capable of binding to a promoter. By combining the polypeptide as shown in SEQ ID NO: 50 with another polypeptide and allowing expression of the protein in a host at high levels, the effects of promoter activation can be attained. In addition to the amino acid sequence as shown in SEQ ID NO: 50, it may be a polypeptide consisting of an amino acid sequence derived from the amino acid sequence as shown in SEQ ID NO: 50 by substitution, deletion, insertion, and/or addition of one or a plurality of amino acids or an amino acid sequence having 85% or higher, 90% or higher, 95% or higher, and more preferably 99% or higher sequence identity with the amino acid sequence as shown in SEQ ID NO: 50. The number of amino acids defined by the term "a plurality of amino acids" is not particularly limited, provided that the functions of the MPP1 homolog are retained. The number of amino acids is up to 6, more preferably up to 5, further preferably up to 4, and most preferably up to 3 or 2.

In the present invention, the term "expression" refers to transcription or translation of a nucleotide sequence that causes production of a protein, which is a gene product. Such expression may be constant regardless of conditions such as external stimulus or growth conditions, or it may be influenced by such conditions. A promoter that activates expression is not particularly limited, provided that such promoter activates expression of a nucleotide sequence encoding a target protein.

The term "high-level expression" used in the present invention refers to an increased protein level in a host or culture solution or an increased mRNA level in the host, compared with a general level. For example, the protein or mRNA level may be measured using an antibody that recognizes a protein or by a technique such as RT-PCR, Northern hybridization, or hybridization using DNA arrays, and the determined level may be compared with that of a non-modified strain, such as a parent strain or wild-type strain. Thus, high-level expression can be confirmed.

The high-level expression of the MPP1 homolog can be realized by conventional techniques, such as modification of the MPP1 homolog gene of the chromosome and/or the vector by increase of the copy number, insertion of a promoter, or modification of a codon.

A strain expressing the MPP1 homolog gene at high levels via introduction of a mutation into the host can be obtained via conventional techniques, such as mutation of the host with the aid of a drug or ultraviolet rays, followed by selection of a strain expressing the MPP1 homolog at high levels.

The term "protein" used in the present invention is composed of two or more amino acids joined by peptide bonds, including short-chain proteins generally referred to as peptides or oligopeptides and long-chain proteins referred to as polypeptides.

The "target protein" of the present invention is not particularly limited, and it can be, for example, an enzyme derived from a microorganism or a protein produced by a multicellular organism, such as an animal or plant. Examples thereof include phytase, protein A, protein G, protein L, amylase, glucosidase, cellulase, lipase, protease, glutaminase, peptidase, nuclease, oxidase, lactase, xylanase, trypsin, pectinase, isomerase, and fluorescent protein. Preferable examples include human and/or animal therapeutic proteins.

Specific examples of human and/or animal therapeutic proteins include hepatitis B virus surface antigen, hirudin, antibody, human antibody, partial antibody, human partial antibody, serum albumin, human serum albumin, epidermal growth factor, human epidermal growth factor, insulin, growth hormone, erythropoietin, interferon, antihemophilic factor, granulocyte colony-stimulating factor (G-CSF), granulocyte-macrophage colony-stimulating factor (GM-CSF), thrombopoietin, IL-1, IL-6, tissue plasminogen activator (TPA), urokinase, leptin, and stem cell growth factor (SCF).

The term "antibody" refers to a heterotetrameric protein composed of two L- and H-polypeptide chains joined by disulfide bonds. An antibody is not particularly limited, provided that it is capable of binding to a particular antigen.

The term "partial antibody" refers to Fab antibody, (Fab)₂ antibody, scFv antibody, diabody antibody, or a derivative of any thereof. A partial antibody is not particularly limited, provided that it is capable of binding to a particular antigen. The term "Fab antibody" refers to a heteromeric protein comprising the L-chain and the Fd chain of the antibody joined by S-S bonds, or a heteromeric protein comprising the L-chain and the Fd chain of the antibody joined with each other without S-S bonds. A heteromeric protein is not particularly limited, provided that it is capable of binding to a particular antigen.

An amino acid constituting a protein may be a naturally occurring, non-natural, or modified amino acid. The amino acid sequence of a protein may be artificially modified or de novo designed.

In the present invention, the term "capacity for producing a target protein" refers to the capacity for producing a target protein in a host cell or the potential that can be achieved via introduction of a gene encoding such protein.

The term "yeast for transformation" used in the present invention refers to a yeast capable of producing a target protein at high levels via introduction of a gene encoding the target protein. Such yeast expresses PaMPP1, PpMPP1, a polypeptide comprising the amino acid sequence as shown in SEQ ID NO: 49, or a polypeptide comprising the amino acid sequence as shown in SEQ ID NO: 50 at high levels.

Examples of techniques of yeast transformation include known techniques such as electroporation, the lithium acetate method, and the spheroplast method, although the techniques are not limited thereto. For example, *Pichia angusta* is generally transformed via electroporation as described in "Highly-efficient electrotransformation of the yeast *Hansenula polymorpha*" (Curr. Genet., 25: 305.310.).

Expression of the "transcription factor" may be enhanced, in addition to that of the factor that activates the aforementioned promoter in the yeast for transformation of the present invention. The term "transcription factor" used in the present invention refers to a substance that activates the initiation of transcription or protein production by a promoter in a mechanism for protein production by a promoter.

The high-level expression of the transcription factor can be realized by conventional techniques, such as modification of the transcription factor gene of the chromosome and/or the vector by increase of the number of copies, insertion of a promoter, or modification of a codon.

A strain expressing the transcription factor gene at high levels via introduction of a mutation into the host can be obtained via conventional techniques, such as mutation of the host with the aid of a drug or ultraviolet rays, followed by selection of a strain expressing the transcription factor gene at high levels.

A preferable transcription factor acts additively or synergistically with the MPP1 homolog, although a transcription factor is not limited thereto. An example of a transcription factor is Mut3 derived from *Pichia angusta.* Mut3 is a polypeptide with the GenBank Accession Number AY046886, the sequence of which is as shown in SEQ ID NO: 46.

The transcription factor of the present invention may be a homolog of Mut3 isolated from another yeast cell. An example thereof is Trm1 of *Candida boidinii.*

Mut3 of the present invention is preferably a polypeptide consisting of the amino acid sequence as shown in SEQ ID NO: 46. Alternatively, it may be a polypeptide consisting of an amino acid sequence derived from the amino acid sequence as shown in SEQ ID NO: 46 by substitution, deletion, insertion, and/or addition of one or a plurality of amino acids or an amino acid sequence having 85% or higher, 90% or higher, 95% or higher, and more preferably 99% or higher sequence identity with the amino acid sequence as shown in SEQ ID NO: 46. The number of amino acids defined by the term "a plurality of amino acids" is not particularly limited, provided that functions of the transcription factor are retained. The number of amino acids is up to 139, more preferably up to 130, further preferably up to 120, and most preferably up to 110, 100, 90, 80, 70, 60, 50, 40, 30, 20, 10, 5, 4, 3, or 2.

The MPP1 homolog gene or transcription factor gene used in the present invention may be DNA encoding an MPP1 homolog or transcription factor. Preferable DNA encoding an MPP1 homolog is as shown in SEQ ID NO: 10 or 11 in the Sequence Listing, and preferable DNA encoding a transcription factor is as shown in SEQ ID NO: 12 in the Sequence Listing. Alternatively, DNA encoding an MPP1 homolog or transcription factor may encompass a nucleotide sequence encoding a polypeptide hybridizing under stringent conditions to a nucleotide sequence complementary to the nucleotide sequence as shown in SEQ ID NO: 10, 11, or 12 in the Sequence Listing and have functions as an MPP1 homolog or transcription factor. Further, DNA may encompass a nucleotide sequence having 85% or higher, preferably 90% or higher, more preferably 95% or higher, and further preferably 99% or higher sequence identity with the nucleotide sequence as shown in SEQ ID NO: 10, 11, or 12 and may encode a polypeptide having functions as an MPP1 homolog or transcription factor.

The "DNA hybridizing under stringent conditions" can be obtained by, for example, conducting hybridization in the presence of 0.7 to 1.0 M NaCl at 65°C using a filter on which colony- or plaque-derived DNA is immobilized and washing the filter with a 2x SSC solution (a 1x SSC solution comprises 150 mM sodium chloride and 15 mM sodium citrate) at 65°C. Such DNA can be obtained by washing the filter, preferably with a 0.5x SSC solution at 65°C, more preferably with a 0.2x SSC solution at 65°C, and further preferably with a 0.1x SSC solution at 65°C.

According to the present invention, nucleotide sequence identity can be determined by a method well known in the art using sequence analysis software or the like. An example is a homology search conducted with the use of genetic information processing software (GENETYX Ver. 10, network edition, Genetyx).

In the present invention, yeasts are selected from *Pichia angusta, Pichia pastoris, Pichia methanolica,* and *Pichia minuta.*

*Pichia angusta* is also referred to as *Hansenula polymorpha. Pichia minuta* is also referred to as *Ogataea minuta.*

In general, "methanol-assimilating yeasts" can be cultured with the use of methanol as a sole carbon source. However, the ability to utilize methanol may be lost due to artificial modification or mutation.

Examples of preferable *Pichia angusta* strains include, but are not particularly limited to, NCYC495 (ATCC14754), 8V (ATCC34438), and DL-1 (ATCC26012). Such strains can be obtained from the American Type Culture Collection or other institutions. Strains derived from such strains can also be used, and examples of leucine auxotrophs include NCYC495-derived BY4329, 8V-derived BY5242, and DL-1-derived BY5243. These strains can be distributed by the National BioResource Project. In the present invention, strains derived from the strains mentioned above can also be used.

Examples of preferable *Pichia pastoris* strains include, but are not particularly limited to, Y-11430 and X-33. Such strains can be obtained from the Northern Regional Research Laboratory or other institutions. Strains derived from such strains can also be used. In the present invention, strains derived from the strains mentioned above can also be used.

### 2. Method for protein production

An example of a method for producing a protein using the yeast for transformation of the present invention is a method comprising culturing the yeast for transformation and allowing a protein to accumulate in the yeast or a culture supernatant. While a method involving the use of methanol-assimilating yeast is described as a preferable example, methods are not limited thereto.

Yeasts for transformation can be generally cultured with the use of any medium containing a nutrient source that can be utilized by methanol-assimilating yeasts. For example, a common medium obtained by adequately mixing carbon sources, such as sugars (e.g., glucose, sucrose, and maltose), organic acids (e.g., lactic acid, acetic acid, citric acid, and propionic acid), alcohols (e.g., methanol, ethanol, and glycerol), carbohydrates (e.g., paraffin), fats and oils (e.g., soybean oil and rapeseed oil), or mixtures of any thereof, nitrogen sources, such as ammonium sulfate, ammonium phosphate, urea, yeast extract, meat extract, peptone, and corn steep liquor, and other nutrient sources, such as inorganic salts and vitamins, can be used, and culture can be carried out in a batch or continuous system.

When culturing the yeast for transformation, a single type of carbon source may be used, or a mixture of two or more types of carbon sources may be used. Culture may be initiated in the presence of a carbon source, or a carbon source may be added during culture.

The yeast for transformation may be subjected to culture alone or in combinations of two or more. The yeast for transformation may be subjected to culture in the presence of other microorganisms.

In the present invention, culture can be carried out under general conditions. For example, culture can be carried out at a pH of 2.5 to 10.0 between 10°C and 48°C in an aerobic environment for 10 hours to 10 days.

Proteins produced with the use of the yeast for transformation of the present invention may be present in a culture supernatant, or proteins may be accumulated in the yeast. Alternatively, proteins may be isolated therefrom via any technique. Proteins can be isolated from a culture supernatant or yeast by performing known protein purification techniques in adequate combination. For example, the transformants are first cultured in an adequate medium, and a culture solution is subjected to centrifugation or filtration to remove yeasts from the culture supernatant. The obtained culture supernatant is then subjected to techniques such as salting-out (e.g., ammonium sulfate precipitation or sodium phosphate precipitation), solvent precipitation (e.g., protein fractional precipitation with acetone or ethanol), dialysis, gel filtration chromatography, ion-exchange chromatography, hydrophobic chromatography, affinity chromatography, reverse phase chromatography, or ultrafiltration. Any such techniques may be carried out alone or in combination, and the proteins of the present invention are isolated from the culture supernatant.

The isolated proteins can be used without processing. Alternatively, such proteins may be subjected to pharmacological modification such as PEGylation, modification for the purpose of impartation of enzyme or isotope functions, or other types of modification before use. Also, proteins may be used in various forms of formulations.

When the high-level expression of the MPP1 homolog or transcription factor is to be attained via introduction of the MPP1 homolog gene or the transcription factor gene into a host, it is preferable that an expression vector containing such genes be constructed and the resulting vector then be introduced into a host.

The term "expression vector" used in the present invention refers to a nucleic acid molecule having a function of expressing a target gene in a transformed host cell. The expression vector has, for example, an expression cassette, a homologous recombination region, a selection marker gene, such as an auxotrophic complementary gene or drug tolerant gene, and an autonomously replicating sequence. The vector after transformation may be integrated into the chromosome, or it may be present in the form of an autonomously replicating vector. Examples of autonomously replicating vectors include the YEp vector, the YRp vector, and the YCp vector. In the case of the genus *Pichia,* examples of vectors include, but are not particularly limited to, the pPICHOLI, pHIP, pHRP, and pHARS vectors.

The "expression cassette" is composed of the MPP1 homolog gene and/or the transcription factor gene as promoter activating factors and a promoter to express such genes. The expression cassette may comprise a terminator gene. Accordingly, the expression cassettes include a cassette comprising the MPP1 homolog gene as a promoter activating factor (i.e., the MPP1 homolog gene expression cassette), a cassette comprising the transcription factor gene (i.e., the transcription factor gene expression cassette), and a cassette comprising both the MPP1 homolog gene and the transcription factor gene. The expression cassette can be constructed in a plasmid such as pUC19, or it can be prepared via PCR.

A promoter used for MPP1 homolog gene expression is not particularly limited, and a promoter that can induce gene expression with a selected carbon source may adequately be used.

When a carbon source is methanol, examples of promoters for MPP1 homolog gene expression include, but are not particularly limited to, the MOX promoter, the FMD promoter, the DHAS promoter, the AOX promoter, and the GAP promoter.

When a carbon source is glucose or glycerol, examples of promoters for MPP1 homolog gene expression include, but are not particularly limited to, the GAP promoter, the TEF promoter, the LEU2 promoter, the URA3 promoter, the ADE promoter, the ADH1 promoter, and the PGK1 promoter.

For transcription factor gene expression, a promoter that can induce gene expression with a selected carbon source may adequately be used, and the promoters for MPP1 homolog gene expression exemplified above can also serve as promoters for transcription factor gene expression.

The "target protein expression vector" of the present invention comprises a "target protein expression cassette" having a promoter activated by the MPP1 homolog, and preferably the methanol-inducible promoter, in the 5' side of the target protein gene and the termination codon and/or the terminator sequence in the 3' side of the target protein gene. When the expression vector is to be integrated into the chromosome, the vector preferably comprises a nucleotide sequence having 50% or higher sequence identity with a 50-bp or longer nucleotide sequence of the yeast chromosome.

When a target protein is to be secreted from the yeast, a nucleotide sequence encoding a signal sequence may be introduced into the 5' end of the target protein gene. A nucleotide sequence encoding a signal sequence is not particularly limited, provided that such nucleotide sequence encodes a signal sequence that can be secreted and expressed by yeast. Examples thereof include nucleotide sequences encoding signal sequences of the mating factor α (MFα) of *Saccharomyces cerevisiae,* acid phosphatase (PHO1) of *Pichia angusta,* acid phosphatase (PHO1) of *Pichia pastoris,* invertase (SUC2) of *Saccharomyces cerevisiae,* PLB1 of *Saccharomyces cerevisiae,* bovine serum albumin (BSA), human serum albumin (HSA), and immunoglobulin.

The "target protein expression cassette", "MPP1 homolog gene expression cassette", "transcription factor gene expression cassette", and the like of the present invention may be contained in a single type of vector. In such a case, specifically, a yeast host and a transformant are produced simultaneously.

When the MPP1 homolog gene, the transcription factor gene, and the target protein-encoding gene are introduced into yeast, use of selection marker genes, such as the auxotrophic complementary gene or drug tolerant gene, together with the MPP1 homolog gene expression cassette, the transcription factor gene expression cassette, and the target protein gene cassette, is preferable. A selection marker is not particularly limited. Auxotrophic complementary genes, such as the URA3 gene, the LEU2 gene, the ADE1 gene, or the HIS4 gene, can be selected by restoration of the prototrophic phenotypes of the uracil-, leucine-, adenine-, or histidine-auxotrophs, respectively. Drug tolerant genes, such as the G418 tolerant gene, the zeocin tolerant gene, or the hygromycin tolerant gene, can be selected based on the tolerance in a medium containing G418, zeocin, or hygromycin, respectively. The auxotrophic selection marker used when preparing a yeast host cannot be used, unless the selection marker is not destroyed. In such a case, the selection marker may be restored by methods known in the art.

When the MPP1 homolog gene or transcription factor gene is to be integrated into the chromosome, it is preferable to prepare such genes so as to have DNA comprising homologous regions of the yeast chromosome. The MPP1 homolog expression cassette or transcription factor gene expression cassette may be integrated into the chromosome via non-specific integration without using homologous regions, integration using a single homologous region, or double-integration using two homologous regions.

Regarding homologous regions, there may be 50% or higher sequence identity between the nucleotide sequence of the chromosome and that of the expression vector. While the length thereof is not particularly limited, it is preferably 50 bp or longer. Sequence identity between homologous regions is more preferably 70% or higher, further preferably 80% or higher, still further preferably 90% or higher, and most preferably 100%.

In the case of integration using a homologous region, for example, a homologous region of the plasmid vector is cleaved at one or more sites with restriction enzymes, so as to locate the homologous region at the end of a linear expression vector. Thus, transformation can be achieved. In the case of homologous integration into the MOX terminator, for example, a linear expression vector obtained by cleaving the MOX terminator sequence at the *Nru*I site or the *Eco*RV site may be used to integrate the expression cassette into the MOX terminator.

The number of copies of expression cassettes per chromosome is not particularly limited. The location in the chromosome at which the MPP1 homolog gene or transcription factor gene expression vector has been integrated is not particularly limited, provided that the MPP1 homolog gene or the transcription factor gene is produced in the strain. When two or more copies of expression vectors are integrated into a transformant, a plurality of expression vectors may be integrated thereinto at the same position, or they may be integrated at different positions one by one.

Methods for preparing the target protein gene, the MPP1 homolog gene, or the transcription factor gene of the present invention are not particularly limited. Such genes may be prepared via PCR or other techniques from microorganism cells, yeast cells, insect cells, plant cells, mammalian animal cells, or phage display libraries. Alternatively, the target gene may be fully synthesized based on such nucleotide sequence. For example, a DNA primer designed based on the MPP1 homolog gene is synthesized, chromosome DNA is isolated from a yeast having the MPP1 homolog gene, and PCR is carried out using the chromosome DNA as a template and the above DNA primer. Thus, the target gene can be obtained.

The target protein gene, MPP1 homolog gene, or transcription factor gene on the chromosome can be modified by introducing a gene into a host chromosome via homologous recombination, site-directed mutagenesis, or other techniques. For example, the protein gene can be introduced into the chromosome so as to increase the number of copies, a promoter located upstream of the protein may be substituted with a more potent promoter, or a codon can be modified to become more suitable as a host of the protein gene.

### Examples

Hereafter, the present invention is described in greater detail with reference to the examples, although the present invention is not limited thereto. The recombinant DNA technology employed in the examples below is specifically described in the following books: Molecular Cloning 2nd Edition, Cold Spring Harbor Laboratory Press, 1989; and Current Protocols in Molecular Biology, Greene Publishing Associates and Wiley-Interscience.

Plasmids obtained in the examples below were amplified using transformants obtained with the use of *E. coli* DH5α competent cells (Takara Bio Inc.) under the designated conditions.

The MOX promoter (SEQ ID NO: 1), the MOX terminator (SEQ ID NO: 2), and the LEU2 gene (SEQ ID NO: 3) used when constructing an antibody expression vector were prepared by PCR using, as a template, genomic DNA of *Pichia angusta* 8V. The AOX promoter (SEQ ID NO: 4) was prepared by PCR using genomic DNA of *Pichia pastoris* X-33 as a template. The mating factor α pre-pro signal gene (MFα, SEQ ID NO: 5) was prepared by PCR using genomic DNA of *Saccharomyces cerevisiae* S288c as a template.

The antibody gene was prepared by PCR using, as templates, the L-chain gene (SEQ ID NO: 6) and the Fd-chain gene (SEQ ID NO: 7) (JP 2009-082033 A) chemically synthesized based on the disclosed sequence information of the fully humanized anti-TNF-a antibody (adalimumab; HUMIRA®).

In addition, the GAP promoter used for vector construction (SEQ ID NO: 8), the G418 tolerant gene regulated by the GAP promoter (SEQ ID NO: 9), the PaMPP1 gene (SEQ ID NO: 10), the PpMPP1 gene (SEQ ID NO: 11), and the Mut3 gene (SEQ ID NO: 12) were obtained by PCR using, for example, genomic DNA of *Pichia angusta* NCYC495, genomic DNA of *Pichia pastoris* X-33, or an adequate commercially available vector as templates.

PCR was carried out using the Prime STAR HS DNA Polymerase (Takara Bio Inc.) in accordance with the manufacturer's instructions. Genomic DNAs were prepared from yeasts using Dr. GenTLE® (Takara Bio Inc.) in accordance with the manufacturer's instructions.

Plasmids were prepared by transformation into *E. coli* DH5α competent cells (Takara Bio Inc.). Plasmids were prepared from plasmid-carrying strains using the QIAprep spin miniprep kit (QIAGEN).

### (Example 1) Construction of fully humanized anti-TNF-α Fab antibody expression vector

A gene fragment (SEQ ID NO: 13) having the *Hind*III*-Not*I*-Bam*HI*-Spe*I*-Mun*I*-Bg*III-*Xba*I-*Eco*RI multicloning sites was fully synthesized, and the resultant was inserted into the *Hind*III and *Eco*RI sites of pUC19 to prepare pUC-1. Also, a gene fragment having the *Hind*III recognition sequences on both sides of the LEU2 gene was prepared by PCR using the primers 1 and 2 (SEQ ID NOs: 14 and 15), the fragment was treated with *Hind*III, and it was then inserted into the *Hind*III site of pUC-1 to prepare pUC-2. Subsequently, a gene fragment having the *Bam*HI recognition sequences on both sides of the MOX promoter was prepared by PCR using the primers 3 and 4 (SEQ IDs NO: 16 and 17), the fragment was treated with *Bam*HI*,* and it was then inserted into the *Bam*HI site of pUC-2 to prepare pUC-Pmox. A gene fragment having the *Eco*RI recognition sequences on both sides of the AOX promoter was prepared by PCR using the primers 5 and 6 (SEQ ID NOs: 18 and 19), the fragment was treated with *Eco*RI*,* and it was then inserted into the *Mun*I site of pUC-2 to prepare pUC-Paox.

A gene fragment having the *Mun*I recognition sequences on both sides of the MOX promoter was prepared by PCR using the primers 7 and 8 (SEQ ID NOs: 20 and 21), the fragment was treated with *Mun*I, and it was then inserted into the *Mun*I site of pUC-Pmox to prepare pUC-PmoxPmox. A gene fragment having the *Bam*HI recognition sequences on both sides of the AOX promoter was prepared by PCR using the primers 9 and 10 (SEQ ID NOs: 22 and 23), the fragment was treated with *Bam*HI*,* and it was then inserted into the *Bam*HI site of pUC-Paox to prepare pUC-PaoxPaox. A gene fragment having the *Xba*I recognition sequences on both sides of the MOX promoter was prepared by PCR using the primers 11 and 12 (SEQ ID NOs: 24 and 25), the fragment was treated with *Xba*I, and it was then inserted into the *Xba*I sites of pUC-PmoxPmox and pUC-PaoxPaox to prepare pUC-PmoxPmoxTm and pUC-PaoxPaoxTm.

A gene fragment having the *SpeI* recognition sequence in a site upstream of MFα was prepared by PCR using the primers 13 and 14 (SEQ ID NOs: 26 and 27). A gene fragment having the 19-bp 3'-terminal fragment of MFα in a site upstream of the L-chain and the *SpeI* recognition sequence in a site downstream of the L-chain was prepared by PCR using the primers 15 and 16 (SEQ ID NOs: 28 and 29). These gene fragments were mixed to prepare a template, and PCR was carried out using the resulting template and the primers 13 and 16 to prepare a gene fragment having *SpeI* recognition sequences on both sides of a fusion gene of MFα and the L-chain. The resulting gene fragment was treated with *Spe*I and inserted into the *SpeI* sites of pUC-PmoxPmoxTm and pUC-PaoxPaoxTm to prepare pUC-PmoxLPmoxTm and pUC-PaoxLPaoxTm.

Separately, a gene fragment having the *Bgl*II recognition sequence in a site upstream of MFα was prepared by PCR using the primers 17 (SEQ ID NO: 30) and 14. A gene fragment having the 19-bp 3'-terminal fragment of MFα in a site upstream of the Fd-chain and the *Bgl*II recognition sequence in a site downstream of the Fd-chain was prepared by PCR using the primers 18 and 19 (SEQ ID NOs: 31 and 32). These gene fragments were mixed to prepare a template, and PCR was carried out using the resulting template and the primers 17 and 19 to prepare a gene fragment having the *Bgl*II recognition sequences on both sides of a fusion gene of MFα and the Fd-chain. The resulting gene fragment was treated with *Bgl*II and inserted into the *Bgl*II sites of pUC-PmoxLPmoxTm and pUC-PaoxLPaoxTm to prepare pUC-PmoxLPmoxFdTm and pUC-PaoxLPaoxFdTm.

pUC-PmoxLPmoxFdTm is designed to allow expression of the L-chain and the Fd-chain of the Fab antibody under the control of the MOX promoter. pUC-PaoxLPaoxFdTm is designed to allow expression of the L-chain and the Fd-chain of the Fab antibody under the control of the AOX promoter.

### (Example 2) Construction of PaMPP1 expression vector

A gene fragment having the *Xba*I recognition sequences on both sides of the MOX terminator was prepared by PCR using the primers 11 and 12, the fragment was treated with *Xba*I, and it was then inserted into the *Xba*I site of pUC-1 prepared in Example 1 to prepare pUCTm.

Subsequently, a gene fragment having the *Eco*RI recognition sequences on both sides of the GAP promoter was prepared by PCR using the primers 20 and 21 (SEQ ID NOs: 33 and 34), the fragment was treated with *Eco*RI*,* and it was then inserted into the *Mun*I site of pUCTm to prepare pUCPgapTm. Subsequently, a gene fragment having the *Eco*RI recognition sequences on both sides of the G418 tolerant gene controlled by the GAP promoter was prepared by PCR using the primers 20 and 22 (SEQ ID NO: 35), the fragment was treated with *Eco*RI*,* and it was then inserted into the *Eco*RI site of pUCPgapTm to prepare pUCPgapTmG418. Subsequently, a gene fragment having the *Bam*HI recognition sequences on both sides of the PaMPP1 gene was prepared by PCR using the primers 23 and 24 (SEQ ID NOs: 36 and 37), the fragment was treated with *Bam*HI*,* and it was then inserted into the *Bgl*II site of pUCPgapTmG418 to construct pUCPgapPaMPP1 TmG418.

This expression vector is designed to allow expression of PaMPP1 (SEQ ID NO: 38) under the control of the GAP promoter.

### (Example 3) Construction of PpMPP1 expression vector

A gene fragment having the *Bam*HI recognition sequences on both sides of the PpMPP1 gene was prepared by PCR using the primers 25 and 26 (SEQ ID NOs: 39 and 40), the fragment was treated with *Bam*HI*,* and it was then inserted into the *Bgl*II site of pUCPgapTmG418 to construct pUCPgapPpMPP1TmG418.

This expression vector is designed to allow expression of PpMPP1 (SEQ ID NO: 41) under the control of the GAP promoter.

### (Example 4) Construction of Mut3 expression vector

A gene fragment having the *Eco*RI recognition sequences on both sides of the G418 tolerant gene controlled by the GAP promoter was prepared by PCR using the primers 20 and 22, the fragment was treated with *Eco*RI*,* and it was then inserted into the *Eco*RI site of pUCTm to prepare pUCTmG418. Subsequently, a gene fragment having the *Spe*I recognition sequence on 5' terminal side of the Mut3 gene and *Bam*HI recognition sequence on 3' terminal side the Mut3 gene was prepared by PCR using the primers 27 and 28 (SEQ ID NOs: 42 and 43), the fragment was treated with *Spe*I and *Bam*HI*,* and the resultant was inserted into the *Spe*I and *Bgl*II sites of pUCTmG418 to construct pUCMut3TmG418. Subsequently, a gene fragment having the *Bam*HI recognition sequences on both sides of the GAP promoter was prepared by PCR using the primers 29 and 30 (SEQ ID NOs: 44 and 45), the fragment was treated with *Bam*HI*,* and it was then inserted into the *Bam*HI site of pUCMut3TmG418 to construct pUCPgapMut3TmG418.

This expression vector is designed to allow expression of Mut3 (SEQ ID NO: 46) under the control of the GAP promoter.

### (Example 5) Construction of PaMPP1/Mut3 co-expression vector

A gene fragment having the *Bam*HI recognition sequence on 5' terminal side of the Mut3 gene and the *Spe*I recognition sequence on 3' terminal side of the Mut3 gene was prepared by PCR using the primers 31 and 32 (SEQ ID NOs: 47 and 48), the fragment was treated with *Bam*HI and *Spe*I, and the resultant was inserted into the *Bam*HI and *Spe*I sites of pUCPgapPaMPP1TmG418 obtained in Example 2 to construct pUCMut3PgapPaMPP1TmG418. Subsequently, a gene fragment having the *Bam*HI recognition sequences on both sides of the GAP promoter was prepared by PCR using the primers 29 and 30, the fragment was treated with *BamHI,* and it was then inserted into the *Bam*HI site of pUCMut3PgapPaMPP1TmG418 to construct pUCPgapMut3PgapPaMPP1TmG418.

This expression vector is designed to allow co-expression of PaMPP1 and Mut3 under the control of the GAP promoter.

### (Example 6) Construction of PaMPP1/Mut3/PpMPP1 co-expression vector

A gene fragment having the *Not*I recognition sequence on 5' terminal side of the PpMPP1 gene and the *Eco5*2I recognition sequence on 3' terminal side of the PpMPP1 gene was prepared by PCR using pUCPgapPpMPP1TmG418 obtained in Example 3 as a template and the primers 33 and 34 (SEQ ID NOs: 51 and 52). The fragment was treated with *Not*I and *Eco*52I*,* and it was then inserted into the *Not*I site of pUCPgapMut3PgapPaMPP1TmG418 obtained in Example 5 to construct pUCPgapPpMPP1PgapMut3PgapPaMPP1TmG418.

This expression vector is designed to allow co-expression of PaMPP1, Mut3, and PpMPP1 under the control of the GAP promoter.

### (Example 7) Preparation of MPP1 homolog-expressing host yeast using MPP1 homolog expression vector

*E. coli* strains were transformed with the use of pUCPgapPaMPP1TmG418, pUCPgapPpMPP1TmG418, pUCPgapMut3TmG418, pUCPgapMut3PgapPaMPP1TmG418, and pUCPgapPpMPP1PgapMut3PgapPaMPP1TmG418 constructed in Examples 2 to 6, the resulting transformants were cultured in 5 ml of 2YT medium (1.6% Bacto Tryptone (Difco), 1.0% Bacto Yeast Extract (Difco), and 0.5% NaCl), and pUCPgapPaMPP1TmG418, pUCPgapPpMPP1TmG418, pUCPgapMut3TmG418, pUCPgapMut3PgapPaMPP1TmG418, and pUCPgapPpMPP1PgapMut3PgapPaMPP1TmG418 were obtained from the resulting strains using the QIAprep spin miniprep kit (QIAGEN).

The plasmids were cleaved at the *Eco*RV or *Nru*I site in the MOX terminator gene or the *Not*I site in the multicloning site to prepare linearized plasmids. With the use of such linearized pUCPgapPaMPP1TmG418, pUCPgapPpMPP1TmG418, pUCPgapMut3TmG418, pUCPgapMut3PgapPaMPP1TmG418, and pUCPgapPpMPP1PgapMut3PgapPaMPP1TmG418, *Pichia angusta* and *Pichia pastoris* were subjected to transformation in the manner described below.

The leucine-auxotrophs of *Pichia angusta* NCYC495 were inoculated into 3 ml of YPD medium (1% Bacto Yeast Extract (Difco), 2% peptone, and 2% glucose) and subjected to shake culture at 37°C overnight to obtain a pre-culture solution. The obtained pre-culture solution (500 µl) was inoculated into 50 ml of YPD medium, shake culture was conducted at 37°C until OD 600 reached 1 to 1.5, the culture product was centrifuged at 3,000 x g and 20°C for 10 minutes to harvest cells, and the cells were resuspended in 10 ml of 50 mM potassium phosphate buffer (pH 7.5) containing 250 µl of 1M DTT (final concentration: 25 mM).

The suspension was incubated at 37°C for 15 minutes, the cells were harvested by centrifugation at 3,000 x g and 20°C for 10 minutes, and the resultant was washed with 50 ml of STM buffer (270 mM sucrose, 10 mM Tris-HCl, and 1 mM magnesium chloride; pH 7.5), which had been ice-cooled in advance. After the cells were harvested by centrifugation at 3,000 x g and 4°C for 10 minutes, the cells were washed again with 25 ml of ice-cooled STM buffer, followed by centrifugation at 3,000 x g and 4°C for 10 minutes to harvest cells. The cells were suspended in 250 µl of ice-cooled STM buffer at the end, and the resulting suspension was designated as a competent cell solution.

The competent cell solution (60 µl) was mixed with 3 µl of a solution of linearized pUCPgapPaMPP1TmG418, pUCPgapPpMPP1TmG418, pUCPgapMut3TmG418, pUCPgapMut3PgapPaMPP1TmG418, or pUCPgapPpMPP1PgapMut3PgapPaMPP1TmG418, the mixtures were transferred into an electroporation cuvette (a disposable cuvette; electrode gap: 2 mm; BM Equipment Co., Ltd.) and pulsed at 7.5 kV/cm, 10 µF, and 900 Ω, the resultant was suspended in 1 ml of YPD medium, and the suspension was then allowed to stand at 37°C for 1 hour. Thereafter, cells were harvested by centrifugation at 3,000 x g at room temperature for 5 minutes, the harvested cells were suspended in 1 ml of YNBMSG medium (0.17% yeast nitrogen base w/o amino acid and ammonium sulfate (Difco) and 0.1% sodium glutamate), and the cells were harvested from the suspension again by centrifugation at 3,000 x g at room temperature for 5 minutes. The cells were resuspended in an adequate amount of YNBMSG medium, the resulting cell suspension was applied to the YNBMSGG418 selection agar plate (0.17% yeast nitrogen base w/o amino acid and ammonium sulfate (Difco), 0.1% sodium glutamate, 1.5% agarose, 2% glucose, 0.05% G418 disulfate, and 100 mg/l leucine), and the cells grown as a result of stationary culture at 37°C for 3 days were selected. Thus, 5 types of *Pichia angustan MPP1* homolog-expressing host yeast cells were obtained.

The leucine-auxotrophs of *Pichia pastoris* X-33 were inoculated into 3 ml of YPD medium (1% Bacto Yeast Extract (Difco), 2% peptone, and 2% glucose) and subjected to shake culture at 30°C overnight to obtain a pre-culture solution. The obtained pre-culture solution (500 µl) was inoculated into 100 ml of YPD medium, shake culture was conducted at 30°C until OD 600 reached 1 to 1.5, cells were harvested by centrifugation at 1,500 x g and 4°C for 5 minutes, and the harvested cells were resuspended in 100 ml of sterile water.

The suspension was centrifuged at 1,500 x g and 4°C for 5 minutes to harvest cells, and the harvested cells were resuspended in 50 ml of sterile water. The suspension was centrifuged at 1,500 x g and 4°C for 5 minutes to harvest cells, and the cells were resuspended in 4 ml of a 1 M sorbitol solution. The resulting suspension was centrifuged at 1,500 x g and 4°C for 5 minutes to harvest cells, the cells were resuspended in 200 µl of a 1 M sorbitol solution, and the resulting suspension was designated as the competent cell solution.

The competent cell solution (50 µl) was mixed with 3 µl of a solution of linearized pUCPgapPaMPP1TmG418, pUCPgapPpMPP1TmG418, pUCPgapMut3TmG418, or pUCPgapMut3PgapPaMPP1TmG418, the mixtures were transferred into an electroporation cuvette (a disposable cuvette; electrode gap: 2 mm; BM Equipment Co., Ltd.) and pulsed at 7.5 kV/cm, 25 µF, and 200 Ω, the resulting cells were suspended in 1 ml of a 1 M sorbitol solution, the resulting cell suspension was applied to the YNBMSGG418 selection agar plate (0.17% yeast nitrogen base w/o amino acid and ammonium sulfate (Difco), 0.1% sodium glutamate, 1.5% agarose, 2% glucose, 0.05% G418 disulfate, and 100 mg/l leucine), and the cells grown as a result of stationary culture at 30°C for 3 days were selected. Thus, 4 types of *Pichia pastoris* MPP1 homolog-expressing host yeast cells were obtained.

### (Example 8) Preparation of transformant expressing MPP1 homolog and fully humanized anti-TNF-a Fab antibody using fully humanized anti-TNF-a Fab antibody expression vector

*E. coli* strains were transformed with the use of pUC-PmoxLPmoxFdTm and pUC-PaoxLPaoxFdTm constructed in Example 1, the transformants were cultured in 5 ml of 2YT medium (1.6% Bacto Tryptone (Difco), 1.0% Bacto Yeast Extract (Difco), and 0.5% NaCl), and pUC-PmoxLPmoxFdTm and pUC-PaoxLPaoxFdTm were obtained from the cells using the QIAprep spin miniprep kit (QIAGEN).

The plasmids were cleaved at the *Eco*RV site in the MOX terminator gene to prepare linearized plasmids. With the use of such linearized pUC-PmoxLPmoxFdTm and pUC-PaoxLPaoxFdTm, *Pichia angusta* was transformed with pUC-PmoxLPmoxFdTm, and *Pichia pastoris* was transformed with pUC-PaoxLPaoxFdTm in the manner described below.

The 5 types of *Pichia angustan MPP1* homolog-expressing host yeast cells obtained in Example 7 or the leucine-auxotrophs of *Pichia angusta* NCYC495 were inoculated into 3 ml of YPD medium (1% Bacto Yeast Extract (Difco), 2% peptone, and 2% glucose) and subjected to shake culture at 37°C overnight to obtain a pre-culture solution. The obtained pre-culture solution (500 µl) was inoculated into 50 ml of YPD medium, shake culture was conducted at 37°C until OD 600 reached 1 to 1.5, cells were harvested by centrifugation at 3,000 x g and 20°C for 10 minutes, and the harvested cells were resuspended in 10 ml of 50 mM potassium phosphate buffer (pH 7.5) containing 250 µl of 1M DTT (final concentration: 25 mM).

The suspension was incubated at 37°C for 15 minutes, followed by centrifugation at 3,000 x g and 20°C for 10 minutes to harvest cells and washing with 50 ml of STM buffer (270 mM sucrose, 10 mM Tris-HCl, 1 mM magnesium chloride; pH 7.5), which had been ice-cooled in advance. After the wash solution was centrifuged at 3,000 x g and 4°C for 10 minutes to harvest cells, the cells were washed again with 25 ml of ice-cooled STM buffer, followed by centrifugation at 3,000 x g and 4°C for 10 minutes to harvest cells. The cells were suspended in 250 µl of ice-cooled STM buffer at the end, and the resulting suspension was designated as a competent cell solution.

The competent cell solution (60 µl) was mixed with 3 µl of a solution of linearized pUC-PmoxLPmoxFdTm, the mixture was transferred into an electroporation cuvette (a disposable cuvette; electrode gap: 2 mm; BM Equipment Co., Ltd.) and pulsed at 7.5 kV/cm, 10 µF, and 900 Ω, the resulting cells were suspended in 1 ml of YPD medium, and the suspension was then allowed to stand at 37°C for 1 hour. Thereafter, the suspension was centrifuged at 3,000 x g at room temperature for 5 minutes to harvest cells, the cells were suspended in 1 ml of YNBMSG medium (0.17% yeast nitrogen base w/o amino acid and ammonium sulfate (Difco) and 0.1% sodium glutamate), and the resultant was centrifuged again at 3,000 x g at room temperature for 5 minutes to harvest cells. The cells were resuspended in an adequate amount of YNBMSG medium, transformants of *Pichia angustan MPP1 homolog-expressing* host yeast cells were applied to the YNBMSGG418 selection agar plate (0.17% yeast nitrogen base w/o amino acid and ammonium sulfate (Difco), 0.1% sodium glutamate, 1.5% agarose, 2% glucose, and 0.05% G418 disulfate), the leucine-auxotrophic transformants of *Pichia angusta* NCYC495 were applied to the YNB selection agar plate (0.67% yeast nitrogen base w/o amino acid (Difco), 1.5% agarose, and 2% glucose), and the cells grown as a result of stationary culture at 37°C for 3 days were selected. Thus, transformants of *Pichia angusta* expressing MPP1 homolog and fully humanized anti-TNF-a Fab antibody and transformants of *Pichia angusta* expressing fully humanized anti-TNF-a Fab antibody were obtained.

The four types of MPP1 homolog-expressing host yeast cells of *Pichia pastoris* obtained in Example 7 or the leucine-auxotrophs of *Pichia pastoris* X-33 were inoculated into 3 ml of YPD medium (1% Bacto Yeast Extract (Difco), 2% peptone, and 2% glucose) and subjected to shake culture at 30°C overnight to obtain a pre-culture solution. The obtained pre-culture solution (500 µl) was inoculated into 100 ml of YPD medium, shake culture was conducted at 30°C until OD 600 reached 1 to 1.5, cells were harvested by centrifugation at 1,500 x g and 4°C for 5 minutes, and the harvested cells were resuspended in 100 ml of sterile water.

The suspension was centrifuged at 1,500 x g and 4°C for 5 minutes to harvest cells, and the cells were resuspended in 50 ml of sterile water. The suspension was centrifuged at 1,500 x g and 4°C for 5 minutes to harvest cells, and the cells were resuspended in 4 ml of a 1 M sorbitol solution. The resulting suspension was centrifuged at 1,500 x g and 4°C for 5 minutes to harvest cells, the cells were resuspended in 200 µl of a 1 M sorbitol solution, and the suspension was designated as the competent cell solution.

The competent cell solution (50 µl) was mixed with 3 µl of a solution of linearized pUC-PaoxLPaoxFdTm, the mixture was transferred into an electroporation cuvette (a disposable cuvette; electrode gap: 2 mm, BM Equipment Co., Ltd.) and pulsed at 7.5 kV/cm, 25 µF, and 200 Ω, the resulting cells were suspended in 1 ml of a 1 M sorbitol solution, the transformants of *Pichia pastoris* (as MPP1 homolog-expressing host cells) were applied to the YNBMSGG418 selection agar plate (0.17% yeast nitrogen base w/o amino acid and ammonium sulfate (Difco), 0.1% sodium glutamate, 1.5% agarose, 2% glucose, and 0.05% G418 disulfate), the leucine-auxotrophic transformants of *Pichia pastoris* were applied to the YNB selection agar plate (0.67% yeast nitrogen base w/o amino acid (Difco), 1.5% agarose, and 2% glucose), and the strains grown as a result of stationary culture at 30°C for 3 days were selected. Thus, transformants of *Pichia pastoris* expressing MPP1 homolog and fully humanized anti-TNF-a Fab antibody and transformants of *Pichia pastoris* expressing fully humanized anti-TNF-a Fab antibody were obtained.

### (Example 9) Culture of transformants expressing MPP1 homolog and fully humanized anti-TNF-a Fab antibody

The transformants expressing MPP1 homolog and fully humanized anti-TNF-a Fab antibody and the transformants expressing fully humanized anti-TNF-a Fab antibody obtained in Example 8 were inoculated into 2 ml of BMGY medium (1% Bacto Yeast Extract, 2% peptone, 0.34% yeast nitrogen base w/o amino acid and ammonium sulfate (Difco), 1% ammonium sulfate, 0.4 mg/l biotin, 100 mM potassium phosphate (pH 6.0), and 2% glycerol) and subjected to shake culture at 37°C and 120 rpm for 72 hours, and the culture supernatant was then recovered by centrifugation at 15,000 rpm and 4°C for 1 minute.

### (Example 10) Measurement of amount of fully humanized anti-TNF-a Fab antibody secretion by ELISA

The amount of the anti-TNF-a Fab antibody secreted and expressed in the culture supernatant of each of the transformants expressing MPP1 homolog and fully humanized anti-TNF-a Fab antibody was measured by sandwich ELISA (i.e., enzyme-linked immunosorbent assay) in the manner described below. The goat anti-human IgG (Fab-specific) antibody (SIGMA) diluted 2,500-fold with an immobilization buffer (a 0.1 N sodium bicarbonate solution; pH 9.6) was applied to an ELISA plate (MaxiSoap, Nunc) at 50 µl/well, and the plate was subjected to incubation at 4°C overnight.

After the completion of incubation, the solution was removed from the wells, the wells were blocked with 250 µl of Immunoblock (Dainippon Sumitomo Pharma Co., Ltd.), and the plate was allowed to stand at room temperature for 1 hour. The product was washed three times with PBST buffer (8 g/l sodium chloride, 0.2 g/l potassium chloride, 1.15 g/l monosodium phosphate (anhydrous), 0.2 g/l potassium dihydrogen phosphate (anhydrous), and 0.1% Tween 20), and serial dilutions of the standard Fab antibody (Anti-Human IgG Fab, Rockland) the culture supernatants were added at 50 µl/well, and the reaction was allowed to proceed at room temperature for 1 hour.

After the plate was washed twice with PBST buffer, a secondary antibody solution diluted 8,000-fold with PBST buffer (secondary antibody: Anti-human IgG (Fab-specific)-HRP conjugate antibody (SIGMA)) was added at 50 µl/well, and the reaction was allowed to proceed at room temperature for 1 hour. After the plate was washed four times with PBST buffer, 100 µl of TMB-1 Component Microwell Peroxidase Substrate SureBlue (KPL) was added, and the reaction solution was allowed to stand at room temperature for 20 minutes. The reaction was terminated with the addition of 100 µl of TMB Stop Solution (KPL), and the absorbance at 450 nm was then measured using a microplate reader (BenchMark Plus, Bio-Rad). The Fab antibody in the culture supernatant was quantified using the calibration curve for the standard Fab antibody. The amount of secretory expression of the anti-TNF-a Fab antibody determined by the above method is shown in Fig. 1.

As a result, the amounts of antibody secretory expression attained with the transformants expressing PaMPP1 (pUCPgapPaMPP1TmG418-introduced) and fully humanized anti-TNF-α Fab antibody, the transformants expressing PpMPP1 (pUCPgapPpMPP1TmG418-introduced) and fully humanized anti-TNF-α Fab antibody, and the transformants expressing Mut3 (pUCPgapMut3TmG418-introduced) and fully humanized anti-TNF-α Fab antibody were found to be apparently higher than those attained with the host strains (i.e., transformants expressing fully humanized anti-TNF-α Fab antibody).

Further, the amounts of antibody secretory expression attained with the transformants co-expressing PaMPP1/Mut3 (pUCPgMut3PgMPP1TmG418-introduced) and fully humanized anti-TNF-α Fab antibody were significantly higher than those attained with the hosts (i.e., transformants expressing fully humanized anti-TNF-α Fab antibody) and those attained with the transformants expressing the PaMPP1 (pUCPgMPP1TmG418-introduced) and fully humanized anti-TNF-α Fab antibody. Since the amounts of antibody secretory expression attained with the transformants co-expressing PaMPP1/Mut3/PpMPP1 (pUCPgapPpMPP1PgapMut3PgapPaMPP1TmG418) and fully humanized anti-TNF-α Fab antibody were lower than those attained with the transformants co-expressing PaMPP1/Mut3 and fully humanized anti-TNF-α Fab antibody, a combination of PaMPP1 with Mut3 was found to be effective.

### Industrial Applicability

The present invention can be used in the field of production of proteins and, in particular, in relation to antibodies used for treatment of diseases.

## Claims

1. A yeast suitable for transformation having an improved capacity of producing target protein by high level expression of an MPP1 homolog,
wherein the yeast is selected from (A) or (B):
(A) wherein the yeast is modified to express an MPP1 homolog of (1) or (2) at high levels by increasing the copy number of the gene encoding said MPP1 homolog, by insertion of a promotor or modification of a codon:
(1) wherein the MPP1 homolog is any of the polypeptides (a)-(c) below:
(a) a polypeptide consisting of the amino acid sequence as shown in SEQ ID NO: 38;
(b) a polypeptide consisting of an amino acid sequence derived from the amino acid sequence as shown in SEQ ID NO: 38 by substitution, deletion, insertion, and/or addition of one to 102 amino acids; or
(c) a polypeptide consisting of an amino acid sequence having 85% or higher sequence identity with the amino acid sequence as shown in SEQ ID NO: 38; or
(2) wherein the MPP1 homolog is partially composed of any of the polypeptides (g)-(i) below:
(g) a polypeptide consisting of the amino acid sequence as shown in SEQ ID NO: 49;
(h) a polypeptide consisting of an amino acid sequence derived from the amino acid sequence as shown in SEQ ID NO: 49 by substitution, deletion, insertion, and/or addition of one to 6 amino acids; or
(i) a polypeptide consisting of an amino acid sequence having 85% or higher sequence identity with the amino acid sequence as shown in SEQ ID NO: 49;
and
wherein the yeast is further modified to express a transcription factor according to (3) at high levels by increasing the copy number of the gene encoding said transcription factor, by insertion of a promotor or modification of a codon:
(3) wherein the transcription factor is any of the polypeptides (m), (n), and (o) below:
(m) a polypeptide consisting of the amino acid sequence as shown in SEQ ID NO: 46;
(n) a polypeptide consisting of an amino acid sequence derived from the amino acid sequence as shown in SEQ ID NO: 46 by substitution, deletion, insertion, and/or addition of one to 139 amino acids; and
(o) a polypeptide consisting of an amino acid sequence having 85% or higher sequence identity with the amino acid sequence as shown in SEQ ID NO: 46;
and
wherein the yeast is of the genus *Pichia* and selected from the group consisting of *Pichia angusta, Pichia pastoris, Pichia methanolica,* and *Pichia minuta,*
or
(B) wherein the yeast is modified to express an MPP1 homolog of (1) or (2) at high levels by increasing the copy number of the gene encoding said MPP1 homolog, by insertion of a promotor or modification of a codon:
(1) wherein the MPP1 homolog is any of the polypeptides (d), (e) and (f) below:
(d) a polypeptide consisting of the amino acid sequence as shown in SEQ ID NO: 41;
(e) a polypeptide consisting of an amino acid sequence derived from the amino acid sequence as shown in SEQ ID NO: 41 by substitution, deletion, insertion, and/or addition of one to 133 amino acids; or
(f) a polypeptide consisting of an amino acid sequence having 85% or higher sequence identity with the amino acid sequence as shown in SEQ ID NO: 41; or
(2) wherein the MPP1 homolog is partially composed of any of the polypeptides (j), (k), and (1) below:
(j) a polypeptide consisting of the amino acid sequence as shown in SEQ ID NO: 50;
(k) a polypeptide consisting of an amino acid sequence derived from the amino acid sequence as shown in SEQ ID NO: 50 by substitution, deletion, insertion, and/or addition of one to 6 amino acids; or
(l) a polypeptide consisting of an amino acid sequence having 85% or higher sequence identity with the amino acid sequence as shown in SEQ ID NO: 50
and
wherein the yeast is of the genus *Pichia* and selected from the group consisting of *Pichia angusta, Pichia pastoris, Pichia methanolica,* and *Pichia minuta.*

2. The yeast suitable for transformation according to claim 1 (A), wherein the MPP1 homolog is encoded by the gene consisting of any of the nucleotide sequences (p-1), (q-1), (r-1), and (s-1) below:
(p-1) a nucleotide sequence encoding the MPP1 homolog;
(q-1) the nucleotide sequence as shown in SEQ ID NO: 10;
(r-1) a nucleotide sequence hybridizing under stringent conditions to a nucleotide sequence complementary to the nucleotide sequence as shown in SEQ ID NO: 10; and
(s-1) a nucleotide sequence having 85% or higher sequence identity with the nucleotide sequence as shown in SEQ ID NO: 10.

3. The yeast suitable for transformation according to claim 1(A), wherein the transcription factor is encoded by the gene consisting of any of the nucleotide sequences (t), (u), (v), and (w) below:
(t) a nucleotide sequence encoding Mut3 derived from Pichia angusta or a homolog of Mut3 derived from another yeast cell;
(u) a nucleotide sequence as shown in SEQ ID NO: 12;
(v) a nucleotide sequence hybridizing under stringent conditions to a nucleotide sequence complementary to the nucleotide sequence as shown in SEQ ID NO: 12; and
(w) a nucleotide sequence having 85% or higher sequence identity with the nucleotide sequence as shown in SEQ ID NO: 12.

4. The yeast suitable for transformation according to claim 1 (B), wherein the MPP1 homolog is encoded by the gene consisting of any of the nucleotide sequences (p-2), (q-2), (r-2), and (s-2) below:
(p-2) a nucleotide sequence encoding the MPP1 homolog;
(q-2) the nucleotide sequence as shown in SEQ ID NO: 11;
(r-2) a nucleotide sequence hybridizing under stringent conditions to a nucleotide sequence complementary to the nucleotide sequence as shown in SEQ ID NO: 11; and
(s-2) a nucleotide sequence having 85% or higher sequence identity with the nucleotide sequence as shown in SEQ ID NO: 11.

5. The yeast suitable for transformation according to claims 1 to 4, wherein the expression level of the target protein is controlled by a promoter that is activated by the MPP1 homolog.

6. The yeast suitable for transformation according to claim 5, wherein the promoter is a methanol-inducible promoter.

7. The yeast suitable for transformation according to claim 6, wherein the methanol-inducible promoter is selected from the group consisting of the AMO promoter, the AOX1 promoter, the AOX2 promoter, the CAT promoter, the DAK promoter, the DAO promoter, the DHAS promoter, the FAO promoter, the FDH promoter, the FGH promoter, the FMD promoter, the GAP promoter, the MOX promoter, the MPP1 promoter, the Mut3 promoter, the Mxr1 promoter, the PER6 promoter, the PER10 promoter, the Pex3 promoter, the Pex5 promoter, the Pex8 promoter, the Pex10 promoter, the PHO1 promoter, the Prm1 promoter, the SNF1 promoter, the Swilp promoter, the Trm1 promoter, the Trm2 promoter, the YNA1 promoter, and the YNA2 promoter.

8. The yeast suitable for transformation according to claim 6, wherein the methanol-inducible promoter is selected from the group consisting of the AOX promoter, the DHAS promoter, the FMD promoter, and the YNA2 promoter.

9. A method for producing a target protein, **characterized by** comprising using the yeast suitable for transformation according to any of claims 1 to 8, wherein the yeast suitable for transformation according to any of claims 1 to 8 is cultured.

10. The method for producing a target protein according to claim 9, wherein culture is conducted using glucose, and/or glycerol, and/or methanol as a carbon source.

11. The method for producing a target protein according to claim 9, wherein culture is conducted using glucose, or glycerol, or methanol as a carbon source.

## Patentansprüche

1. Für Transformation geeignete Hefe, welche eine erhöhte Kapazität für die Produktion von Zielprotein durch ein hohes Expressionslevel eines MPP1-Homologs hat,
wobei die Hefe aus (A) oder (B) ausgewählt wird:
(A) wobei die Hefe modifiziert ist, um ein MPP1-Homolog gemäß (1) oder (2), durch Erhöhen der Kopienzahl des das MPP1-Homolog codierenden Gens, auf einem hohen Level zu exprimieren, durch Einfügen eines Promotors oder Modifikation eines Codons:
(1) wobei das MPP1-Homolog irgendeines der folgenden Polypeptide (a)-(c) ist:
(a) ein Polypeptid, bestehend aus der wie in SEQ ID NO: 38 gezeigten Aminosäuresequenz;
(b) ein Polypeptid, bestehend aus einer Aminosäuresequenz, abgeleitet von der wie in SEQ ID NO: 38 gezeigten Aminosäuresequenz durch Substitution, Deletion, Einfügen und/oder Hinzufügen von einer bis zu 102 Aminosäure(n); oder
(c) ein Polypeptid, bestehend aus einer Aminosäuresequenz, welche eine 85% oder höhere Sequenzidentität mit der wie in SEQ ID NO: 38 gezeigten Aminosäuresequenz aufweist; oder
(2) wobei das MPP1-Homolog teilweise aus den irgendeinem der folgenden Polypeptide (g)-(i) zusammengesetzt ist:
(g) ein Polypeptid, bestehend aus der wie in SEQ ID NO: 49 gezeigten Aminosäuresequenz;
(h) ein Polypeptid, bestehend aus einer Aminosäuresequenz, abgeleitet von der wie in SEQ ID NO: 49 gezeigten Aminosäuresequenz durch Substitution, Deletion, Einfügen und/oder Hinzufügen von einer bis zu 6 Aminosäure(n); oder
(i) ein Polypeptid, bestehend aus einer Aminosäuresequenz, welche eine 85% oder höhere Sequenzidentität mit der wie in SEQ ID NO: 49 gezeigten Aminosäuresequenz aufweist;
und
wobei die Hefe weitergehend modifiziert ist um einen Transkriptionsfaktor gemäß (3), durch Erhöhen der Kopienzahl des den Transkriptionsfaktor codierenden Gens, auf einem hohen Level zu exprimieren, durch Einfügen eines Promotors oder Modifikation eines Codons:
(3) wobei der Transkriptionsfaktor irgendeiner der folgenden Polypeptide (m), (n) und (o) ist:
(m) ein Polypeptid, bestehend aus der wie in SEQ ID NO: 46 gezeigten Aminosäuresequenz;
(n) ein Polypeptid, bestehend aus einer Aminosäuresequenz, abgeleitet von der wie in SEQ ID NO: 46 gezeigten Aminosäuresequenz durch Substitution, Deletion, Einfügen und/oder Hinzufügen von einer bis zu 139 Aminosäure(n); und
(o) ein Polypeptid, bestehend aus einer Aminosäuresequenz, welche eine 85% oder höhere Sequenzidentität mit der wie in SEQ ID NO: 46 gezeigten Aminosäuresequenz aufweist;
und
wobei die Hefe vom Genus *Pichia* ist, und ausgewählt wird aus der Gruppe bestehend aus *Pichia angusta, Pichia pastoris, Pichia methanolica* und *Pichia minuta,* oder
(B) wobei die Hefe modifiziert ist, um ein MPP1-Homolog gemäß (1) oder (2), durch Erhöhen der Kopienzahl des das MPP1-Homolog codierenden Gens, auf einem hohen Level zu exprimieren, durch Einfügen eines Promotors oder Modifikation eines Codons:
(1) wobei das MPP1-Homolog irgendeines der folgenden Polypeptide (d), (e) und (f) ist:
(d) ein Polypeptid, bestehend aus der wie in SEQ ID NO: 41 gezeigten Aminosäuresequenz;
(e) ein Polypeptid, bestehend aus einer Aminosäuresequenz, abgeleitet von der wie in SEQ ID NO: 41 gezeigten Aminosäuresequenz durch Substitution, Deletion, Einfügen und/oder Hinzufügen von einer bis zu 133 Aminosäure(n); oder
(f) ein Polypeptid, bestehend aus einer Aminosäuresequenz, welche eine 85% oder höhere Sequenzidentität mit der wie in SEQ ID NO: 41 gezeigten Aminosäuresequenz aufweist; oder
(2) wobei das MPP1-Homolog teilweise aus irgendeinem der folgenden Polypeptide (j), (k) und (1) zusammengesetzt ist:
(j) ein Polypeptid, bestehend aus der wie in SEQ ID NO: 50 gezeigten Aminosäuresequenz;
(k) ein Polypeptid, bestehend aus einer Aminosäuresequenz, abgeleitet von der wie in SEQ ID NO: 50 gezeigten Aminosäuresequenz durch Substitution, Deletion, Einfügen und/oder Hinzufügen von einer bis zu 6 Aminosäure(n); oder
(1) ein Polypeptid, bestehend aus einer Aminosäuresequenz, welche eine 85% oder höhere Sequenzidentität mit der wie in SEQ ID NO: 50 gezeigten Aminosäuresequenz aufweist;
und
wobei die Hefe vom Genus *Pichia* ist, und ausgewählt wird aus der Gruppe bestehend aus *Pichia angusta, Pichia pastoris, Pichia methanolica* und *Pichia minuta.*

2. Für Transformation geeignete Hefe gemäß Anspruch 1 (A), wobei das MPP1-Homolog von dem Gen, bestehend aus irgendeiner der folgenden Nukleotidsequenzen (p-1), (q-1), (r-1) und (s-1), codiert wird:
(p-1) eine Nukleotidsequenz, welche das MPP1-Homolog codiert;
(q-1) die wie in SEQ ID NO: 10 gezeigte Nukleotidsequenz;
(r-1) eine Nukleotidsequenz, die unter stringenten Bedingungen an eine Nukleotidsequenz, die komplementär zur wie in SEQ ID NO: 10 gezeigten Nukleotidsequenz ist, hybridisiert; und
(s-1) eine Nukleotidsequenz, welche 85% oder höhere Sequenzidentität mit der wie in SEQ ID NO: 10 gezeigten Nukleotidsequenz aufweist.

3. Für Transformation geeignete Hefe gemäß Anspruch 1 (A), wobei der Transkriptionsfaktor von dem Gen, bestehend aus irgendeiner der folgenden Nukleotidsequenzen (t), (u), (v) und (w), codiert wird:
(t) eine Nukleotidsequenz, welche von Pichia angusta abgeleitetes Mut3 oder ein Homolog eines von einer anderen Hefezelle abgeleiteten Mut3 codiert;
(u) eine wie in SEQ ID NO: 12 gezeigte Nukleotidsequenz;
(v) eine Nukleotidsequenz, die unter stringenten Bedingungen an eine Nukleotidsequenz, die komplementär zur wie in SEQ ID NO: 12 gezeigten Nukleotidsequenz ist, hybridisiert; und
(w) eine Nukleotidsequenz, welche 85% oder höhere Sequenzidentität mit der wie in SEQ ID NO: 12 gezeigten Nukleotidsequenz aufweist.

4. Für Transformation geeignete Hefe gemäß Anspruch 1 (B), wobei das MPP1-Homolog von dem Gen, bestehend aus irgendeiner der folgenden Nukleotidsequenzen (p-2), (q-2), (r-2) und (s-2), codiert wird:
(p-2) eine Nukleotidsequenz, welches das MPP1-Homolog codiert;
(q-2) die wie in SEQ ID NO: 11 gezeigte Nukleotidsequenz;
(r-2) eine Nukleotidsequenz, die unter stringenten Bedingungen an eine Nukleotidsequenz, die komplementär zur wie in SEQ ID NO: 11 gezeigten Nukleotidsequenz ist, hybridisiert; und
(s-2) eine Nukleotidsequenz, welche 85% oder höhere Sequenzidentität mit der wie in SEQ ID NO: 11 gezeigten Nukleotidsequenz aufweist.

5. Für Transformation geeignete Hefe gemäß Ansprüchen 1 bis 4, wobei das Expressionslevel des Zielproteins durch einen Promotor kontrolliert wird, welcher durch das MPP1-Homolog aktiviert wird.

6. Für Transformation geeignete Hefe gemäß Anspruch 5, wobei der Promotor ein Methanol-induzierbarer Promotor ist.

7. Für Transformation geeignete Hefe gemäß Anspruch 6, wobei der Methanol-induzierbare Promotor aus gewählt wird aus der Gruppe bestehend aus dem AMO-Promotor, dem AOX1-Promotor, dem AOX2-Promotor, dem CAT-Promotor, dem DAK-Promotor, dem DAO-Promotor, dem DHAS-Promotor, dem FAO-Promotor, dem FDH-Promotor, dem FGH-Promotor, dem FMD-Promotor, dem GAP-Promotor, dem MOX-Promotor, dem MPP1-Promotor, dem Mut3-Promotor, dem Mxr1-Promotor, dem PER6-Promotor, dem PER10-Promotor, dem Pex3-Promotor, dem Pex5-Promotor, dem Pex8-Promotor, dem Pex10-Promotor, dem PHO1-Promotor, dem Prm1-Promotor, dem SNF1-Promotor, dem Swi1p-Promotor, dem Trm1-Promotor, dem Trm2-Promotor, dem YNA1-Promotor und dem YNA2-Promotor.

8. Für Transformation geeignete Hefe gemäß Anspruch 6, wobei der Methanol-induzierbare Promotor ausgewählt wird aus der Gruppe bestehend aus dem AOX-Promotor, dem DHAS-Promotor, dem FMD-Promotor und dem YNA2-Promotor.

9. Verfahren zur Produktion eines Zielproteins, charakterisiert durch Umfassen der Verwendung der für Transformation geeigneten Hefe gemäß irgendeinem der Ansprüche 1 bis 8, wobei die für Transformation geeignete Hefe gemäß irgendeinem der Ansprüche 1 bis 8 kultiviert wird.

10. Verfahren zur Produktion eines Zielproteins gemäß Anspruch 9, wobei das Kultivieren unter Verwendung von Glucose und/oder Glycerin und/oder Methanol als Kohlenstoff-Quelle ausgeführt wird.

11. Verfahren zur Produktion eines Zielproteins gemäß Anspruch 9, wobei das Kultivieren unter Verwendung von Glucose oder Glycerin oder Methanol als Kohlenstoff-Quelle ausgeführt wird.

## Revendications

1. Levure appropriée pour une transformation ayant une capacité améliorée de production de protéine cible par l'expression à un niveau élevé d'un homologue de MPP1,
dans laquelle la levure est sélectionnée parmi (A) ou (B) :
(A) dans laquelle la levure est modifiée pour exprimer un homologue de MPP1 de (1) ou (2) à des niveaux élevés par augmentation du nombre de copie du gène codant pour ledit homologue de MPP1, par insertion d'un promoteur ou modification d'un codon :
(1) dans laquelle l'homologue de MPP1 est l'un quelconque des polypeptides (a) à (c) ci-dessous :
(a) un polypeptide consistant en la séquence d'acides aminés représentée par SEQ ID NO : 38 ;
(b) un polypeptide consistant en une séquence d'acides aminés dérivée de la séquence d'acides aminés représentée par SEQ ID NO : 38 par substitution, délétion, insertion, et/ou addition d'un à 102 acides aminés ; ou
(c) un polypeptide consistant en une séquence d'acides aminés présentant une identité de séquence de 85 % ou plus avec la séquence d'acides aminés représentée par SEQ ID NO : 38 ; ou
(2) dans laquelle l'homologue de MPP1 est partiellement composé de l'un quelconque des polypeptides (g) à (i) ci-dessous :
(g) un polypeptide consistant en la séquence d'acides aminés représentée par SEQ ID NO : 49 ;
(h) un polypeptide consistant en une séquence d'acides aminés dérivée de la séquence d'acides aminés représentée par SEQ ID NO : 49 par substitution, délétion, insertion, et/ou addition d'un à 6 acides aminés ; ou
(i) un polypeptide consistant en une séquence d'acides aminés présentant une identité de séquence de 85 % ou plus avec la séquence d'acides aminés représentée par SEQ ID NO : 49 ;
et
dans laquelle la levure est modifiée en outre pour exprimer un facteur de transcription selon (3) à des niveaux élevés par augmentation du nombre de copie du gène codant pour ledit facteur de transcription, par insertion d'un promoteur ou modification d'un codon :
(3) dans laquelle le facteur de transcription est l'un quelconque des polypeptides (m), (n), et (o) ci-dessous :
(m) un polypeptide consistant en la séquence d'acides aminés représentée par SEQ ID NO : 46 ;
(n) un polypeptide consistant en une séquence d'acides aminés dérivée de la séquence d'acides aminés représentée par SEQ ID NO : 46 par substitution, délétion, insertion, et/ou addition d'un à 139 acides aminés ; et
(o) un polypeptide consistant en une séquence d'acides aminés présentant une identité de séquence de 85 % ou plus avec la séquence d'acides aminés représentée par SEQ ID NO: 46;
et
dans laquelle la levure est du genre *Pichia* et est sélectionnée dans le groupe constitué de *Pichia angusta, Pichia pastoris, Pichia methanolica,* et *Pichia minuta,*
ou
(B) dans laquelle la levure est modifiée pour exprimer un homologue de MPP1 de (1) ou (2) à des niveaux élevés par augmentation du nombre de copie du gène codant pour ledit homologue de MPP1, par insertion d'un promoteur ou modification d'un codon :
(1) dans laquelle l'homologue de MPP1 est l'un quelconque des polypeptides (d), (e) et (f) ci-dessous :
(d) un polypeptide consistant en la séquence d'acides aminés représentée par SEQ ID NO : 41 ;
(e) un polypeptide consistant en une séquence d'acides aminés dérivée de la séquence d'acides aminés représentée par SEQ ID NO : 41 par substitution, délétion, insertion, et/ou addition d'un à 133 acides aminés ; ou
(f) un polypeptide consistant en une séquence d'acides aminés présentant une identité de séquence de 85 % ou plus avec la séquence d'acides aminés représentée par SEQ ID NO : 41 ; ou
(2) dans laquelle l'homologue de MPP1 est partiellement composé de l'un quelconque des polypeptides (j), (k), et (l) ci-dessous :
(j) un polypeptide consistant en la séquence d'acides aminés représentée par SEQ ID NO : 50;
(k) un polypeptide consistant en une séquence d'acides aminés dérivée de la séquence d'acides aminés représentée par SEQ ID NO : 50 par substitution, délétion, insertion, et/ou addition d'un à 6 acides aminés ; ou
(l) un polypeptide consistant en une séquence d'acides aminés présentant une identité de séquence de 85 % ou plus avec la séquence d'acides aminés représentée par SEQ ID NO : 50
et
dans laquelle la levure est du genre *Pichia* et est sélectionnée dans le groupe constitué de *Pichia angusta, Pichia pastoris, Pichia methanolica,* et *Pichia minuta.*

2. Levure appropriée pour une transformation selon la revendication 1 (A), dans laquelle l'homologue de MPP1 est codé par le gène consistant en l'une quelconque des séquences nucléotidiques (p-1), (q-1), (r-1), et (s-1) ci-dessous :
(p-1) une séquence nucléotidique codant pour l'homologue de MPP1 ;
(q-1) la séquence nucléotidique représentée par SEQ ID NO : 10 ;
(r-1) une séquence nucléotidique s'hybridant dans des conditions stringentes à une séquence nucléotidique complémentaire à la séquence nucléotidique représentée par SEQ ID NO : 10 ; et
(s-1) une séquence nucléotidique présentant une identité de séquence de 85 % ou plus avec la séquence nucléotidique représentée par SEQ ID NO : 10.

3. Levure appropriée pour une transformation selon la revendication 1 (A), dans laquelle le facteur de transcription est codé par le gène consistant en l'une quelconque des séquences nucléotidiques (t), (u), (v), et (w) ci-dessous :
(t) une séquence nucléotidique codant pour Mut3 dérivé de *Pichia angusta* ou un homologue de Mut3 dérivé d'une autre cellule de levure ;
(u) une séquence nucléotidique représentée par SEQ ID NO : 12 ;
(v) une séquence nucléotidique s'hybridant dans des conditions stringentes à une séquence nucléotidique complémentaire à la séquence nucléotidique représentée par SEQ ID NO : 12 ; et
(w) une séquence nucléotidique présentant une identité de séquence de 85 % ou plus avec la séquence nucléotidique représentée par SEQ ID NO : 12.

4. Levure appropriée pour une transformation selon la revendication 1 (B), dans laquelle l'homologue de MPP1 est codé par le gène consistant en l'une quelconque des séquences nucléotidiques (p-2), (q-2), (r-2), et (s-2) ci-dessous :
(p-2) une séquence nucléotidique codant pour l'homologue de MPP1 ;
(q-2) la séquence nucléotidique représentée par SEQ ID NO : 11 ;
(r-2) une séquence nucléotidique s'hybridant dans des conditions stringentes à une séquence nucléotidique complémentaire à la séquence nucléotidique représentée par SEQ ID NO : 11 ; et
(s-2) une séquence nucléotidique présentant une identité de séquence de 85 % ou plus avec la séquence nucléotidique représentée par SEQ ID NO : 11.

5. Levure appropriée pour une transformation selon les revendications 1 à 4, dans laquelle le niveau d'expression de la protéine cible est régulé par un promoteur qui est activé par l'homologue de MPP1.

6. Levure appropriée pour une transformation selon la revendication 5, dans laquelle le promoteur est un promoteur inductible par le méthanol.

7. Levure appropriée pour une transformation selon la revendication 6, dans laquelle le promoteur inductible par le méthanol est sélectionné dans le groupe constitué du promoteur AMO, du promoteur AOX1, du promoteur AOX2, du promoteur CAT, du promoteur DAK, du promoteur DAO, du promoteur DHAS, du promoteur FAO, du promoteur FDH, du promoteur FGH, du promoteur FMD, du promoteur GAP, du promoteur MOX, du promoteur MPP1, du promoteur Mut3, du promoteur Mxr1, du promoteur PER6, du promoteur PER10, du promoteur Pex3, du promoteur Pex5, du promoteur Pex8, du promoteur Pex10, du promoteur PHO1, du promoteur Prm1, du promoteur SNF1, du promoteur Swi1p, du promoteur Trm1, du promoteur Trm2, du promoteur YNA1, et du promoteur YNA2.

8. Levure appropriée pour une transformation selon la revendication 6, dans laquelle le promoteur inductible par le méthanol est sélectionné dans le groupe constitué du promoteur AOX, du promoteur DHAS, du promoteur FMD, et du promoteur YNA2.

9. Procédé de production d'une protéine cible, **caractérisé en ce qu'**il comprend l'utilisation de la levure appropriée pour une transformation selon l'une quelconque des revendications 1 à 8, dans lequel la levure appropriée pour une transformation selon l'une quelconque des revendications 1 à 8 est mise en culture.

10. Procédé de production d'une protéine cible selon la revendication 9, dans lequel la culture est effectuée en utilisant du glucose, et/ou du glycérol, et/ou du méthanol comme source de carbone.

11. Procédé de production d'une protéine cible selon la revendication 9, dans lequel la culture est effectuée en utilisant du glucose, ou du glycérol, ou du méthanol comme source de carbone.
